(19) Europäisches Patentamt
European Patent Office
Office européen des brevets

(11) Veröffentlichungsnummer: **0 034 260**
**B1**

(12) # EUROPÄISCHE PATENTSCHRIFT

(45) Veröffentlichungstag der Patentschrift: **20.07.83**

(51) Int. Cl.³: **C 07 C 103/52,** A 61 K 37/02

(21) Anmeldenummer: **81100357.3**

(22) Anmeldetag: **19.01.81**

(54) **Peptide, Verfahren zur Herstellung derselben und diese enthaltende Arzneimittel beziehungsweise Diagnostica.**

(30) Priorität: **18.01.80 HU RI010080**

(43) Veröffentlichungstag der Anmeldung: **26.08.81 Patentblatt 81/34**

(45) Bekanntmachung des Hinweises auf die Patenterteilung: **20.07.83 Patentblatt 83/29**

(84) Benannte Vertragsstaaten: **AT BE CH DE FR GB IT LI NL SE**

(56) Entgegenhaltungen:
**DE-A-2-831-271**
**DE-A-2-831-534**
**US-A-3-915-948**
**US-A-3-923-769**
**US-A-3-923-770**
**US-A-3-976-770**

(73) Patentinhaber: **RICHTER GEDEON VEGYESZETI GYAR R.T., Gyömröi ut 19-21, H-1475 Budapest X (HU)**

(72) Erfinder: **Nyéki, Olga Dipl.-Chem., Ungvár u. 56/g, Budapest XIV. (HU)**
Erfinder: **Kisfaludy, Lajos Dr.Dipl.-Chem., Riadó u.6/A, Budapest II (HU)**
Erfinder: **Kárpáti, Egon, Dr., Krisztina Krt. 35, Budapest XI (HU)**
Erfinder: **Szporny, László, Dr., Szabolcska M. u. 7, Budapest XI (HU)**

(74) Vertreter: **Beszédes, Stephan G. Dr., Münchener Strasse 80a Postfach 1168, D-8060 Dachau (DE)**

**EP 0 034 260 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

# Peptide, Verfahren zur Herstellung derselben und diese enthaltende Arzneimittel beziehungsweise Diagnostica.

Die Erfindung betrifft neue in der 8-Stellung eine α-Hydroxycarbonsäure- oder α-Hydroxycarbonsäureestergruppe aufweisende Peptide, ein Verfahren zu ihrer Herstellung und diese enthaltende Arzneimittel beziehungsweise Diagnostica, insbesondere solche mit Angiotensin-II entgegenwirkender beziehungsweise antagonisierender Wirkung.

Das erste Angiotensin-II-analogon, welches sowohl in vitro als auch in vivo als spezifischer Konkurrenzinhibitor von Angiotensin-II wirkt, wurde im Jahre 1970 beschrieben (vergleiche G. R. Marshal und Mitarbeiter, Proc. Nat. Acad. Sci. USA, 67 [1970], 1 624; P. A. Khairallah und Mitarbeiter, J. Med. Chem. 13 [1970], 181.

Diese Beobachtung hat dann zu ausgedehnten weiteren Forschungsarbeiten Anlass gegeben, welche zur Herstellung von zahlreichen weiteren solchen Angiotensin-II entgegenwirkenden beziehungsweise antagonisierenden Angiotensin-II-analoga führten; solche Produkte boten Möglichkeiten zur Diagnostizierung und gegebenenfalls auch zur therapeutischen Behandlung von bestimmten reninabhängigen Bluthochdruck- beziehungsweise Hypertensionserscheinungen. Von den auf diese Weise hergestellten zahlreichen Angiotensin-II entgegenwirkenden beziehungsweise antagonisierenden Verbindungen ist bereits das L-Sarkosyl-L-arginyl-L-valyl-L-tyrosyl-L-isoleucyl-L-histidyl-L-prolyl-L-alanin ([$Sar^1$, $Ala^8$]-angiotensin-II) unter der Bezeichnung Saralasin in den Verkehr gebracht worden (D. T. Pals und Mitarbeiter: Circ. Res. 29 [1971], 673; dieses Produkt hat sich im Laufe der klinischen Untersuchungen als zur Diagnostizierung (G. Bönner und Mitarbeiter: Dtsch. Med. Wschr. 104 [1979], 432) beziehungsweise zur therapeutischen Behandlung (J. L. Marx: Science 194 [1976], 821) von Bluthochdruck beziehungsweise Hypertensionen verschiedenen Ursprunges geeignet erwiesen. Neuerdings wurde festgestellt, dass die Angiotensin-II-antagonisten auch zur Behandlung der als Folgeerscheinung des renovaskularen Bluthochdruckes auftretenden Herzinsuffizienz geeignet sind (H. Gavras und Mitarbeiter: JAMA 238 [1977], 880).

Die Untersuchung der Zusammenhänge zwischen der Struktur und der biologischen Wirkung der bekannten Angiotensin-II-analoga hat auch nützliche Informationen zur Klärung der agonistischen und antagonistischen Wirkungen geliefert. Im Vordergrund der gegenwärtigen Forschungen steht die Herstellung von solchen Antagonisten, welche längere biologische Halbwertszeichen und keine unerwünschten Nebenwirkungen, wie anfängliche agonistische Wirkungen nach der Verabreichung, zeigen.

Ferner sind aus der deutschen Offenlegungsschrift 28 31 271 Peptide der allgemeinen Formel

X–Arg–Val–Tyr–Ile–His–Pro–Y,

worin

X den Rest einer in der α-Stellung eine Hydroxygruppe enthaltenden aliphatischen Carbonsäure, insbesondere eine Hydroxyacetyl- oder α-Hydroxypropionylgruppe, und

Y den Rest einer aliphatischen α-Aminocarbonsäure, insbesondere eine Leucyl-, Isoleucyl-, Alanyl- oder Threonylgruppe,

bedeuten, und ihre Säureadditionssalze und Komplexe sowie ihre pharmakologischen, insbesondere Angiotensin-II entgegenwirkenden, Wirkungen bekannt.

Weiterhin sind in der deutschen Offenlegungsschrift 28 31 534 Peptide der allgemeinen Formel

X–Arg–Val–Tyr–Ile–His–Pro–Y,

worin

X den Rest einer in der α-Stellung eine Aminooxygruppe enthaltenden aliphatischen Carbonsäure, insbesondere eine Aminooxyacetyl- oder α-Aminooxypropionylgruppe, und

Y den Rest einer aliphatischen α-Aminocarbonsäure, insbesondere eine Leucyl-, Isoleucyl-, Alanyl- oder Threonylgruppe,

bedeuten, und ihre Säureadditionssalze und Komplexe sowie ihre pharmakologischen, insbesondere Angiotensin-II entgegenwirkenden, Wirkungen beschrieben.

Ausserdem ist aus der US-Patentschrift 3 923 769 das Octapeptid L-(N-methyl)Ile–L–Arg–L–Val–L–Tyr–L–Ile–L–His–L–Pro–L–Ile bekannt, wobei seine pharmakologische Wirksamkeit als Angiotensin-II entgegenwirkend ohne Vergleichsversuchsergebnisse angegeben ist.

Ferner ist in der US-Patentschrift 3 923 770 das Octapeptid Dimethylglycyl–L–Arg–L–Val–L–Tyr–L–Ile–L–His–L–Pro–L–Ile beschrieben, wobei seine pharmakologische Wirksamkeit als Angiotensin-II entgegenwirkend wiederum ohne Vergleichsversuchsergebnisse angegeben ist.

Ausserdem betrifft die US-Patentschrift 3 976 770 das Octapeptid Sar–L–Arg–L–Val–L–Tyr–L–Ile–L–His–L–Pro–(OMe)Thr, wobei auch in dieser Druckschrift die pharmakologische Wirksamkeit dieser Verbindung als Angiotensin-II entgegenwirkend ohne Vergleichsversuchsergebnisse angegeben ist.

Schliesslich ist aus der US-Patentschrift 3 915 948 Sarcosyl-L-arginyl-L-valyl-L-tyrosyl-L-valyl-L-histidyl-L-prolin bekannt, wobei auch in dieser Druckschrift die pharmakologische Wirksamkeit von deren Verbindung als Angiotensin-II entgegenwirkend ohne Vergleichsversuchsergebnisse angegeben ist.

Der Erfindung liegt die Aufgabe zugrunde, neue Peptidderivate mit überlegenen pharmakologischen Wirkungen, insbesondere Angiotensin-II entgegenwirkenden beziehungsweise antagonisierenden Wirkungen, ohne unerwünschte Nebenwirkungen, ein Verfahren zur Herstellung derselben und diese Verbindungen enthaltende Arzneimittel zu schaffen.

Das Obige wurde überraschenderweise durch die Erfindung erreicht.

Es wurde nämlich überraschenderweise festgestellt, dass wirksame Konkurrenzinhibitoren beziehungsweise kompetitive Inhibitoren von Angiotensin-II durch Ersatz der Phenylalanineinheit in der 8-Stellung des Moleküles von Angiotensin-II durch eine aliphatische α-Hydroxycarbonsäureeinheit beziehungsweise α-Hydroxycarbonsäureestereinheit und Einbauen einer N-Methylaminosäureeinheit, vorzugsweise Sarkosineinheit, oder einer aliphatischen α-Aminooxy- beziehungsweise α-Hydroxycarbonsäureeinheit, beispielsweise einer solchen nach den deutschen Offenlegungsschriften 28 31 271 beziehungsweise 28 31 534, in die 1-Stellung des Moleküles erzielt werden können.

Gegenstand der Erfindung sind daher Peptide der allgemeinen Formel

X–Arg–Val–Tyr–Ile–His–Pro–Y–O–A I,

worin

X einen Acylrest einer N-Methylaminosäure oder einer in der α-Stellung eine Aminooxygruppe oder eine Hydroxygruppe aufweisenden aliphatischen Carbonsäure, jeweils in der L-Konfiguration im Falle eines asymmetrischen Kohlenstoffatomes,

Arg Rest von L-Arginin,

Val einen Rest von L-Valin,

Tyr einen Rest von L-Tyrosin,

Ile einen Rest von L-Isoleucin,

His einen Rest von L-Histidin,

Pro einen Rest von L-Prolin,

Y einen Rest einer aliphatischen α-Hydroxycarbonsäure mit 1 gegenüber dieser Säure fehlenden Wasserstoffatom ihrer Hydroxygruppe ausser dem Fehlen der Hydroxygruppe der Carboxylgruppe, jeweils in der L-Konfiguration im Falle eines asymmetrischen Kohlenstoffatomes, und

A Wasserstoff oder einen Alkylrest mit 1 bis 5 Kohlenstoffatom(en) darstellen,

sowie ihre Säureadditionssalze und pharmazeutisch brauchbaren Komplexe.

Sämtliche erfindungsgemässen Verbindungen unterscheiden sich von den Verbindungen der deutschen Offenlegungsschriften 28 31 271 und 28 31 534 sowie der US-Patentschriften 3 923 769, 3 923 770, 3 976 770 und 3 915 948 darin, dass sie in der 8-Stellung den Rest einer aliphatischen α-Hydroxycarbonsäure statt des Restes einer aliphatischen α-Aminocarbonsäure aufweisen.

Diejenigen erfindungsgemässen Verbindungen, bei welchen A für einen Alkylrest steht, unterscheiden sich von den Verbindungen der genannten Druckschriften zusätzlich noch darin, dass ihre Carbonsäureeinheit in der 8-Stellung durch einen Alkylrest verestert ist. Von der Verbindung der US-Patentschrift 3 923 770 unterscheiden sich die erfindungsgemässen Verbindungen zusätzlich noch darin, dass sie in der 1-Stellung keinen N,N-Dimethylaminoessigsäurerest, sondern den Acylrest einer N-Methylaminosäure oder sonstigen für X festgelegten anderen Rest aufweisen. Zusätzliche Unterschiede der erfindungsgemässen Verbindungen zur Verbindung der US-Patentschrift 3 915 948 bestehen darin, dass die ersteren Octapeptide sind und die letztere ein Heptapeptid ist und die ersteren in der 5-Stellung den Rest von L-Isoleucin im Gegensatz zur letzteren mit ihrem Rest von L-Valin in der 5-Stellung aufweisen.

Auf Grund dieser Unterschiede stellen die erfindungsgemässen Octapeptide eine neue Klasse von Angiotensin-II entgegenwirkenden Verbindungen, welche bisher nicht beschrieben und somit auch pharmakologisch nicht untersucht wurden, dar. Gegenüber den US-Patentschriften 3 923 769, 3 923 770, 3 976 770 und 3 915 948 ist zu bemerken, dass in diesen nicht einmal ein Vergleich der in ihnen beschriebenen Peptide mit dem damals schon bekannten und angewandten L-Sarkosyl-L-arginyl-L-valyl-L-tyrosyl-L-isoleucyl-L-histidyl-L-prolyl-L-alanin [Saralasin] gemacht worden ist. Daraus aber, dass diese bekannten Octa- beziehungsweise Heptapeptide in den seitdem abgelaufenen 5 bis 6 Jahren am internationalen Markt nicht durchdringen konnten, ist zu schliessen, dass sie mit dem L-Sarkosyl-L-arginyl-L-valyl-L-tyrosyl-L-isoleucyl-L-histidyl-L-prolyl-L-alanin [Saralasin] nicht zu konkurrieren vermochten.

Vorzugsweise ist der Acylrest einer N-Methylaminosäure, für den X stehen kann, der Sarkosylrest.

Ferner ist es bevorzugt, dass der Rest einer aliphatischen α-Hydroxycarbonsäure, für den Y steht, ein solcher mit einem asymmetrischen Kohlenstoffatom in der L-Konfiguration, insbesondere von L-Milchsäure oder L-2-Hydroxy-3-methyl-valeriansäure, ist.

Es ist auch bevorzugt, dass der Alkylrest, für den A stehen kann, ein solcher mit 1 oder 2, ganz besonders 1, Kohlenstoffatom(en) ist.

Besonders bevorzugte erfindungsgemässe Verbindungen sind L-Sarkosyl-L-arginyl-L-valyl-L-tyrosyl-L-isoleucyl-L-histidyl-L-prolyl-L-2-hydroxy-3-methylvaleriansäure und L-Sarkosyl-L-arginyl-L-valyl-L-tyrosyl-L-isoleucyl-L-histidyl-L-prolyl-L-milchsäure sowie ihre Alkylester mit 1 bis 5 Kohlenstoffatomen im Alkylteil.

Unter pharmazeutisch brauchbaren Komplexen der erfindungsgemässen Octapeptide der allgemeinen Formel I sind solche mit organischen oder anorganischen Verbindungen, durch welche eine verzögerte Wirkung beziehungsweise Retardwirkung der Octapeptide gewährleistet ist, zu verstehen. Als organische Verbindungen sind zum Beispiel Gelatine, Carboxymethylcellulosen, Alginsäureester, Poly-(phloretinphosphate), Aminosäurepolymere und -copolymere geeignet. Als anorganische Verbindungen kommen beispielsweise Hydroxyde beziehungsweise schwerlösliche Salze, wie Phosphate, von Metallen, insbesondere von Zink, in Frage.

Gegenstand der Erfindung ist auch ein Verfahren zur Herstellung der erfindungsgemässen Verbindungen, welches dadurch gekennzeichnet ist, dass in der Peptidchemie an sich bekannter Weise eine passende aliphatische α-Hydroxycarbonsäu-

re oder ein Alkylester derselben mit 1 bis 5 Kohlenstoffatomen im Alkylteil oder ein sonstiger Ester derselben nur mit Schutzfunktion der Estergruppe mit der nachfolgend einzubauenden an ihrem endständigen Stickstoffatom eine abspaltbare Schutzgruppe und, soweit erforderlich, an einem gegebenenfalls vorliegenden anderen Stickstoffatom oder Sauerstoffatom eine weitere abspaltbare Schutzgruppe aufweisenden Aminosäure und/oder dem nachfolgend einzubauenden an seinem enständigen Stickstoffatom eine abspaltbare Schutzgruppe und, soweit erforderlich, an einem oder mehreren gegebenenfalls vorliegenden anderen Stickstoff- und/oder Sauerstoffatom(en) eine beziehungsweise mehrere Schutzgruppe(n) aufweisenden Peptidfragment beziehungsweise einem Esterderivat desselben kondensiert wird und gegebenenfalls mit dem erhaltenen am endständigen Stickstoffatom geschützten Peptidzwischenprodukt und dem beziehungsweise den durch etwaige weitere derartige Kondensationen erhaltenen am endständigen Stickstoffatom geschützten weiteren Peptidzwischenprodukt(en) nach in an sich bekannter Weise erfolgendem Entfernen der Schutzgruppe des endständigen Stickstoffatomes eine weitere beziehungsweise weitere Kondensation(en) mit beziehungsweise jeweils mit der nachfolgend einzubauenden an ihrem endständigen Stickstoffatom eine abspaltbare Schutzgruppe und, soweit erforderlich, an einem gegebenenfalls vorliegenden anderen Stickstoffatom oder Sauerstoffatom eine weitere abspaltbare Schutzgruppe aufweisenden Aminosäure und/oder dem nachfolgend einzubauenden an seinem endständigen Stickstoffatom eine abspaltbare Schutzgruppe und, soweit erforderlich, an einem oder mehreren gegebenenfalls vorliegenden anderen Stickstoff- und/oder Sauerstoffatom(en) eine beziehungsweise mehrere Schutzgruppe(n) aufweisenden Peptidfragment beziehungsweise einem Esterderivat desselben vorgenommen wird beziehungsweise werden, wobei so viele Kondensationen durchgeführt werden, wie es zum Einbau aller gewünschten Aminosäureeinheiten erforderlich ist, sowie danach in an sich bekannter Weise vom erhaltenen geschützten Peptidderivat die Schutzgruppe des endständigen Stickstoffatomes und die etwaige(n) Schutzgruppe(n) von anderen Stickstoffatomen beziehungsweise Sauerstoffatomen sowie gegebenenfalls die Aminogruppe der gegebenenfalls vorliegenden Aminooxygruppe selektiv schrittweise oder in einem Schritt entfernt wird beziehungsweise werden, worauf gegebenenfalls in an sich bekannter Weise das erhaltene Peptid der allgemeinen Formel I in ein Säureadditionssalz oder einen Komplex desselben überführt wird beziehungsweise gegebenenfalls das erhaltene Säureadditionssalz des Peptides der allgemeinen Formel I in ein anderes Säureadditionssalz oder in das Peptid der allgemeinen Formel I überführt wird.

Vorzugsweise werden

a) zur Herstellung der Peptide der allgemeinen Formel I, bei welchen A für Wasserstoff steht, durch die Kondensation(en) als geschützte Peptide solche der allgemeinen Formel

B–X–Arg[C]–Val–Tyr[D]–Ile–His[E]–Pro–Y–O–F II,

worin

B für eine durch Acidolyse oder katalytische Hydrogenolyse entfernbare Schutzgruppe, insbesondere eine Benzyloxycarbonyl- oder tert.-Butyloxycarbonylgruppe, steht,

C eine Schutzgruppe zum zeitweiligen Schutz der Guanidinogruppe des Restes von Arginin, insbesondere eine Nitro- oder p-Toluolsulfonylgruppe (Tosylgruppe), bedeutet,

D eine Schutzgruppe zum zeitweiligen Schutz der aromatischen Hydroxygruppe des Restes von Tyrosin, insbesondere eine, gegebenenfalls substituierte, Benzylgruppe, darstellt,

E für eine Schutzgruppe zum zeitweiligen Schutz der Imidazolgruppe des Restes von Histidin, insbesondere eine Dinitrophenylgruppe, steht,

F eine gegen die Einwirkung von milden Säuren widerstandsfähige, aber durch katalytische Hydrogenolyse oder durch die Einwirkung von stärkeren Säuren entfernbare Schutzgruppe zum Schutz der Carboxylgruppe des Restes der C-endständigen α-Hydroxycarbonsäure bedeutet und

X, Arg, Val, Tyr, Ile, His, Pro, Y und A wie oben festgelegt sind, aufgebaut oder

b) zur Herstellung der Peptide der allgemeinen Formel I, bei welchen A einen Alkylrest mit 1 bis 5 Kohlenstoffatomen bedeutet, durch die Kondensation(en) als geschützte Peptide solche der allgemeinen Formel

B–X–Arg[C]–Val–Tyr[D]–Ile–His[E]–Pro–Y–O–A III,

worin

B, C, D und E wie vorstehend festgelegt sind und

X, Arg, Val, Tyr, Ile, His, Pro, Y und A wie oben festgelegt sind, aufgebaut.

Im erfindungsgemässen Verfahren können die Kondensationen zum Aufbau der geschützten Peptidderivate, wie der der allgemeinen Formeln II beziehungsweise III, nach beliebigen in der Peptidchemie üblichen Verfahrensweisen, zum Beispiel nach der in der ungarischen Patentschrift 168 431 beschriebenen Verfahrensweise durch Acylieren der passenden Aminosäuren beziehungsweise α-Hydroxycarbonsäuren beziehungsweise α-Hydroxycarbonsäureester mit Pentafluorphenylestern von an der N-endständigen Aminogruppe durch durch Acidolyse leicht entfernbare Schutzgruppen, wie tert.-Butyloxycarbonylgruppen, geschützten Aminosäuren beziehungsweise Peptiden durchgeführt werden. Dabei sind zum Schutz der seitenfunktionellen Gruppen solche Schutzgruppen, welche unter den Bedingungen der nach dem Acylieren durchzuführenden Entfernung der N-Schutzgruppe, beispielsweise durch Acidolyse, stabil sind, zu verwenden.

Das Einführen der α-Hydroxycarbonsäureeinheit in die 8-Stellung wird zweckmässig in der Weise durchgeführt, dass ein α-Hydroxycarbonsäureester der allgemeinen Formel

H–Y–O–Z IV,

worin

Z für einen Alkyl- oder Aralkylrest steht und

Y wie oben festgelegt ist,

mit an der Aminogruppe, zweckmässig durch eine tert.-Butyloxycarbonylgruppe, geschütztem L-Prolin umgesetzt wird, Die auf diese Weise ausgebildete Carbonyloxygruppe $(-\overset{\overset{O}{\|}}{C}-O-)$ zeigt in den weiteren Schritten der Synthese ein stabiles Verhalten. Soweit der Alkyl- beziehungsweise Aralkylrest, für den Z steht, lediglich eine Schutzfunktion hat, wird er nach dem Aufbau des geschützten Peptidderivates in an sich bekannter Weise abgespalten.

Nach einer vorteilhaften Ausführungsform des erfindungsgemässen Verfahrens werden also die geschützten Peptidderivate, wie die der allgemeinen Formeln II beziehungsweise III, schrittweise aus den einzelnen Aminosäuren beziehungsweise α-Hydroxycarbonsäuren beziehungsweise α-Hydroxycarbonsäureestern aufgebaut. Nach einer anderen Ausführungsform des erfindungsgemässen Verfahrens kann aber auch ein bereits hergestelltes entsprechend geschütztes Teilpeptid auf einmal mit einem anderen ebenfalls bereits hergestelltem Teilpeptid acyliert werden. Diese Verfahrensweise kann zum Beispiel bei der Herstellung der in der 1-Stellung eine α-Aminooxyacylgruppe enthaltenden Peptide vorteilhaft angewandt werden. Zum zeitweiligen Schutz der Aminogruppen der einzelnen Aminosäuren beziehungsweise Aminosäurederivate beziehungsweise Teilpeptide sind bei beiden Ausführungsformen solche Schutzgruppen zu verwenden, welche dann durch bekannte Verfahrensweisen, wie durch Acidolyse, leicht entfernt werden können; besonders die tert.-Butyloxycarbonylgruppe ist für solche Zwekke vorteilhaft anwendbar.

Nach der Beendigung des Aufbaues der geschützten Peptidderivate, wie der der allgemeinen Formeln II beziehungsweise III, werden dann wie bereits erwähnt deren Schutzgruppen abgespalten. Dabei wird zweckmässig in der Weise vorgegangen, dass zunächst die Dinitrophenylschutzgruppe des Restes von Histidin, soweit eine solche vorhanden ist, durch Thiolyse, und zwar vorzugsweise durch Behandlung mit 2-Mercaptoäthanol, abgespalten wird. Die übrigen vorliegenden Schutzgruppen können, soweit eine solche Thiolyse durchgeführt wird, nach ihr, vorteilhaft in einem Schritt, und zwar vorzugsweise durch Acidolyse, insbesondere mit Fluorwasserstoffsäure, oder noch mehr bevorzugt durch katalytische Hydrogenolyse, insbesondere unter Verwendung von Palladium/Aktivkohle als Katalysator, entfernt werden.

Nach einer vorteilhaften Ausführungsform des erfindungsgemässen Verfahrens werden zur Herstellung von Peptiden der allgemeinen Formel I, bei welchen X für einen Acylrest einer in der α-Stellung eine Aminooxygruppe aufweisenden aliphatischen Carbonsäure steht, durch die Kondensation(en) die entsprechenden geschützten Peptide der allgemeinen Formeln II beziehungsweise III, bei welchen X ebenfalls für einen Acylrest einer in der α-Stellung eine Aminooxygruppe aufweisenden aliphatischen Carbonsäure steht, aufgebaut und das Abspalten der Schutzgruppe, für welche B steht, sowie gegebenenfalls der anderen durch Acidolyse abspaltbaren Schutzgruppen von ihnen durch Acidolyse, insbesondere durch Behandeln der, zweckmässig von der Dinitrophenylgruppe befreiten, geschützten Peptidderivate mit Fluorwasserstoffsäure, durchgeführt. Die katalytische Hydrierung kommt hierfür deswegen nicht in Frage, weil durch diese gleichzeitig auch der Acylrest der in der α-Stellung eine Aminooxygruppe aufweisenden aliphatischen Carbonsäure, für den X steht, unter Abspalten der Aminogruppe in den entsprechenden Acylrest der in der α-Stellung eine Hydroxygruppe aufweisenden sonst gleichen aliphatischen Carbonsäure überführt würde.

Nach einer anderen vorteilhaften Ausführungsform des erfindungsgemässen Verfahrens werden zur Herstellung von Peptiden der allgemeinen Formel I, bei welchen X für einen Acylrest einer in der α-Stellung eine Hydroxygruppe aufweisenden aliphatischen Carbonsäure steht, durch die Kondensation(en) die entsprechenden Peptide der allgemeinen Formeln II beziehungsweise III, bei welchen jedoch X für einen Acylrest einer in der α-Stellung eine Aminooxygruppe aufweisenden aliphatischen Carbonsäure steht, aufgebaut und das Abspalten der Schutzgruppe, für welche B steht, sowie gegebenenfalls der anderen durch katalytische Hydrogenolyse abspaltbaren Schutzgruppen von ihnen durch katalytische Hydrierung unter gleichzeitiger Abspaltung der Aminogruppe der genannten α-Aminooxygruppe durchgeführt.

Das gegebenenfalls erfolgende Überführen der erfindungsgemässen Peptide der allgemeinen Formel I in Säureadditionssalze beziehungsweise Komplexe kann nach an sich bekannten Verfahrensweisen durchgeführt werden.

Die erfindungsgemässen Peptide können durch bekannte Verfahrensweisen, zum Beispiel durch Ionenaustauschchromatographie an Carboxymethylcellulose, gereinigt werden. Sie werden zweckmässig in Form von lyophilisierten Pulvern hergestellt, welche sich sehr gut zur Zubereitung von verschiedenen Arzneimittelpräparaten eignen.

Ferner sind erfindungsgemäss Arzneimittel beziehungsweise Diagnostica, welche 1 oder mehr erfindungsgemässe Verbindungen als Wirkstoff beziehungsweise Wirkstoffe, zweckmässigerweise zusammen mit üblichen pharmazeutischen Konfektionierungsmitteln, enthalten, vorgesehen.

Die erfindungsgemässen Verbindungen haben nämlich wie bereits erwähnt wertvolle pharmakologische Wirkungen, insbesondere Angiotensin-II

entgegenwirkende beziehungsweise antagonisierende Wirkungen.

So verringern sie in erheblichem Masse den durch Angiotensin-II herbeigeführten hohen Blutdruck und haben dabei den grossen Vorteil, dass sie diese Wirkung auch bei subkutaner Verabreichung zeigen.

Die Angiotensin-II entgegenwirkende beziehungsweise antagonistische Wirkung von erfindungsgemässen Verbindungen und der anerkannt gut wirksamen Vergleichssubstanz L-Sarkosyl-L-arginyl-L -valyl-L -tyrosyl-L- isoleucyl-L - histidyl-L-prolyl-L -alanin ([$Sar^1$, $Ala^8$]-angiotensin-II) {Saralasin} gleicher Wirkungsrichtung wurde an narkotisierten männlichen Katzen geprüft. Bei den Untersuchungen wurden die Vagusnerven der Tiere an beiden Seiten des Halses durchgeschnitten und nach der Blockierung der Ganglien wurde den Tieren Hypertensin (Ciba) durch Infusion mit einer Geschwindigkeit von 0,5 µg/kg/Minute verabreicht. Nachdem sich der erhöhte Blutdruck stabilisiert hatte, wurde die zu untersuchende Verbindung in physiologischer Kochsalzlösung beziehungsweise in einer Trägerstoffe enthaltenden Lösung intravenös oder subkutan verabreicht. Die darauf eingetretene Blutdrucksenkung in mm Hg wurde in Prozenten des vor der Behandlung gemessenen Blutdruckanfangswertes registriert. Die statistische Auswertung der Messergebnisse erfolgte auf Grund des Student'schen 1-Proben-«t»-Versuches. Als Wirkungsdauer der einzelnen Wirkstoffe wurde die bis zur letzten noch signifikanten Blutdrucksenkung ($p = 5\%$) verstrichene Zeit zugrundegelegt. Die so erhaltenen Versuchsergebnisse sind in der folgenden Tabelle 1 zusammengestellt.

Tabelle 1
Wirkung auf den Blutdruck

| Verbindung | | Verabreichung und deren Wirkung: intravenös | | | | subkutan in physiologischer Kochsalzlösung | | | | subkutan in Carboxymethylcelluloselösung | | | | subkutan in Gelatine lösung | | | |
|---|---|---|---|---|---|---|---|---|---|---|---|---|---|---|---|---|---|
| Beispiel | Bezeichnung | Dosis in µg/kg | Zahl der Versuche | Blutdrucksenkung in % | Zeitdauer in Minuten | Dosis in µg/kg | Zahl der Versuche | Blutdrucksenkung in % | Zeitdauer in Minuten | Dosis in µg/kg | Zahl der Versuche | Blutdrucksenkung in % | Zeitdauer in Minuten | Dosis in µg/kg | Zahl der Versuche | Blutdrucksenkung in % | Zeitdauer in Minuten |
| 1 | L-Sarkosyl-L-arginyl-L-valyl-L-tyrosyl-L-isoleucyl-L-histidyl-L-prolyl-L-milchsäure {[$Sar^1$, L-$Lac^8$]-Ang-II} | 10<br>20 | 5<br>5 | 23<br>29 | 15<br>15 | 50<br>100<br>200 | 5<br>5<br>5 | 29<br>33<br>47 | 90<br>120<br>180 | 50<br>100 | 6<br>6 | 25<br>27 | 45<br>90 | 50<br>100<br>200 | 5<br>5<br>5 | 24<br>32<br>28 | 90<br>90<br>120 |
| 2 | L-Sarkosyl-L-arginyl-L-valyl-L-tyrosyl-L-isoleucyl-L-histidyl-L-prolyl-L-milchsäureäthylester {[$Sar^1$, L-Lac-$OEt^8$]-Ang-II} | 10<br>20 | 5<br>8 | 27<br>40 | 12 | | | | | 100 | 5 | 41 | 90 | | | | |
| 3 | L-Sarkosyl-L-arginyl-L-valyl-L-tyrosyl-L-isoleucyl-L-histidyl-L-prolyl-L-2-hydroxy-3-methylvaleriansäure {[$Sar^1$, $HMV^8$]-Ang-II} | 10<br>20 | 5<br>6 | 33<br>40 | 45<br>20 | | | | | 100 | 5 | 35 | 300 | | | | |
| Vergleichssubstanz | L-Sarkosyl-L-arginyl-L-valyl-L-tyrosyl-L-isoleucyl-L-histidyl-L-prolyl-L-alanin-{Saralasin} | 10 | 5 | 32 | 15 | 100<br>200 | 6<br>5 | 29<br>24 | 60<br>45 | 100<br>200 | 5<br>7 | 23<br>28 | 45<br>90 | 200 | 4 | 23 | keine Dauerwirkung |

Es wurden weitere pharmakologische Versuche nach den folgenden Verfahrensweisen durchgeführt:

a) An isolierten Hauptschlagader- beziehungsweise Aortastreifen von Hasen nach R. F. Furchgott (J. Pharm. Exp. Ther. 108 [1953], 129).

b) Am isolierten Magenfundus von Ratten nach J. R. Vane (Brit. J. Pharm. Chemother. 12 [1957], 344).

c) Hemmung der Aldosteronabsonderung beziehungsweise -sekretion an der isolierten Nebennierenrinde von Ratten (zona glomerulosa) nach J.M. Rossum (Arch. Internat. Pharmacodyn. 143 [1963], 229). Es wurden die $pA_2$-Werte (sie sind Logarithmenzahlen) bestimmt.

Die Ergebnisse dieser Versuche sind in der folgenden Tabelle 2 zusammengestellt.

Tabelle 2

| Verbindung Beispiel | Bezeichnung | Untersuchungsverfahrensweise a) | b) | c) |
|---|---|---|---|---|
| 1 | L-Sarkosyl-L-arginyl-L-valyl-L-tyrosyl-L-isoleucyl-L-histidyl-L-prolyl-L-milchsäure {[$Sar^1$, L-$Lac^8$]-Ang-II} | 11,74 | 12,07 | 8,85 |
| 2 | L-Sarkosyl-L-arginyl-L-valyl-L-tyrosyl-L-isoleucyl-L-histidyl-L-prolyl-L-milchsäureäthylester {[$Sar^1$, L-Lac-$OEt^8$]-Ang-II} | 11,14 | 13,47 | 9,56 |
| 3 | L-Sarkosyl-L-arginyl-L-valyl-L-tyrosyl-L-isoleucyl-L-histidyl-L-prolyl-L-2-hydroxy-3-methyl-valeriansäure {[$Sar^1$, $HMV^8$]-Ang-II} | 12,68 | 11,79 | 8,64 |
| Vergleichssubstanz | L-Sarkosyl-L-arginyl-L-valyl-L-tyrosyl-L-isoleucyl-L-histidyl-L-prolyl-L-alanin {Saralasin} | 11,76 | 11,56 | 8,36 |

Aus den obigen Tabellen 1 und 2 geht hervor, dass die erfindungsgemässen Peptide überlegene Angiotensin-II entgegenwirkende beziehungsweise antagonisierende Wirkungen haben, die sich sowohl in der in vivo ermittelten blutdrucksenkenden Wirkung als auch in den nach den Verfahrensweisen a), b) und c) in vitro ermittelten Wirkungen äussern. Das Mass der blutdrucksenkenden Wirkung ist auch bei subkutaner Verabreichung dieser Verbindungen sehr hoch; in Trägerstoffe enthaltenden Lösungen werden in zahlreichen Fällen Wirkungsdauern von mehreren Stunden erreicht.

Die erfindungsgemässen Peptide einschliesslich ihrer Säureadditionssalze und Komplexe können in Form von üblichen Arzneimittelpräparaten vorliegen und in der Therapie angewandt werden. Diese Arzneimittelpräparate enthalten die erfindungsgemässen Verbindungen in Verbindung mit zur enteralen oder parenteralen Verabreichung geeigneten anorganischen oder organischen Träger- und/oder Hilfsstoffen. Sie können zum Beispiel in Form von festen Lyophilisaten, welche als Trägerstoffe pharmazeutisch brauchbare und mit den erfindungsgemässen Peptiden nicht reagierende feste Stoffe, wie Kohlenhydrate, enthalten, sowie ferner in Form von neben den erfindungsgemässen Peptidwirkstoffen gegebenenfalls auch konservierend oder stabilisierend wirkende Hilfsstoffe enthaltenden verdünnten oder konzentrierten Suspensionen oder Emulsionen vorliegen. Die Zubereitung dieser Arzneimittelpräparateformen kann nach üblichen pharmazeutischen Verfahrensweisen erfolgen.

Die erfindungsgemässen Arzneimittel können zum differenzierenden Diagnostizieren von hohem Blutdruck verschiedenen Ursprunges sowie ferner in der Therapie beispielsweise zum Normalisieren von von den Nieren herrührendem Hohem Blutdruck beziehungsweise Hypertensionen renalen Ursprunges, bei Bluthochdruck- beziehungsweise Hypertensionskrisen und in Fällen von sekundärer Herzinsuffizienz eingesetzt werden.

Die erfindungsgemässen Arzneimittelpräparate liegen zweckmässig in Form von 1 bis 10 mg Wirkstoff enthaltenden Injektionslösungen vor. Die tägliche Dosis der erfindungsgemässen Peptidwirkstoffe beträgt bei erwachsenen Patienten zweckmässig 1 bis 10 mg. Diese Menge wird vorzugsweise auf einmal intravenös, subkutan, intramuskulär oder mit langsamer intravenöser Infusion verabreicht.

Die Erfindung wird an Hand der folgenden Beispiele näher erläutert.

Bei der Herstellung der verschiedenen Verbindungen wurde das Eindampfen der Lösungen jeweils in einem Vakuumeindampfer vom Typ «Rotavapor» durchgeführt. Zur Bestimmung von Schmelzpunkten wurde ein Schmelzpunktmessgerät nach Dr. Tottoli (Büchi) verwendet. Die Dünnschichtchromatogramme wurden auf Silicagelplatten «Kieselgel G nach Stahl» (Merck) hergestellt. Zum Entwickeln der Chromatogramme wurden die folgenden Lösungsmittelgemische verwendet:

| Lösungsmittelgemisch | Volumverhältnis der Lösungsmittel beziehungsweise des vor dem Schrägstrich stehenden Lösungsmittels zum nach dem Schrägstrich stehenden Lösungsmittelgemisch |
|---|---|
| 1. n-Hexan : Äthylacetat | = 4 : 1 |
| 2. Äthylacetat : [Pyridin/Essigsäure/Wasser im Volumverhältnis von 20 : 6 : 11] | = 95 : 5 |
| 3. Äthylacetat : [Pyridin/Essigsäure/Wasser im Volumverhältnis von 20 : 6 : 11] | = 90 : 10 |
| 4. Äthylacetat : [Pyridin/Essigsäure/Wasser im Volumverhältnis von 20 : 6 : 11] | = 80 : 20 |
| 5. Äthylacetat : [Pyridin/Essigsäure/Wasser im Volumverhältnis von 20 : 6 : 11] | = 70 : 30 |
| 6. n-Butanol : Essigsäure : Wasser im Volumverhältnis von 4 : 1 : 5 | [davon wird nur die obere Phase verwendet] |
| 7. n-Butanol : Essigsäure : Pyridin : Wasser | = 30 : 6 : 20 : 24 |
| 8. n-Butanol : Äthylacetat : Essigsäure : Wasser | = 1 : 1 : 1 : 1 |

Das Entwickeln der Flecke erfolgte mit Ninhydrin beziehungsweise mit Chlortolidin/Kaliumjodid.

Zur Reinigung der Endprodukte wurde die folgende allgemeine Verfahrensweise angewandt:

0,5 g freies Peptid wurde in 4 $cm^3$ einer 0,01 m Ammoniumacetatlösung gelöst und dann wurde diese Lösung auf eine 0,5 l Carboxymethylcellulose (CMC-52) enthaltende und vorher mit der obigen Ammoniumacetatpufferlösung ins Gleichgewicht gebrachte Säule geschichtet. Die Säule wurde dann durch Gradienteluieren mit 1,5 l einer 0,01 m Ammoniumacetatlösung und 1,5 l einer 0,4 m Ammoniumacetatlösung, die durch einen Gradientmischer zugeführt wurden, eluiert. Die Strömungsgeschwindigkeit war 25 $cm^3$/Stunde; es wurden Fraktionen von je 10 $cm^3$ aufgefangen. Das von der Säule herabfliessende Eluat wurde mit Hilfe eines Gerätes vom Typ «LKB Uvicord-II» ® (LKB-Produkter AB, Schweden) laufend registriert und dann wurde die auf Grund der erhaltenen Kurve ermittelte Hauptfraktion in einer Lyophilisiereinrichtung vom Typ Leybold-Heraeus lyophilisiert. Erforderlichenfalls wurde das erhaltene Produkt ebenfalls unter Anwendung des Gradienteluierens erneut chromatographiert.

Bei der Aminosäureanalyse bedeuten die Werte in eckigen Klammern jeweils die theoretischen Werte.

Beispiel 1

L-Sarkosyl-L-arginyl-L-valyl-L-tyrosyl-L-isoleucyl-L-histidyl-L-prolyl-L-milchsäure
{[Sarkosin$^1$, L-Milchsäure$^8$]-Angiotensin-II}

1. Stufe

[N-(tert.-Butyloxycarbonyl)]-L-prolyl-L-milchsäurebenzylester

Es wurden 2,15 g (10 Millimol) [N-(tert.-Butyloxycarbonyl)]-L-prolin in 10 $cm^3$ wasserfreiem Tetrahydrofuran gelöst, die Lösung wurde auf 0 °C abgekühlt und es wurden unter Rühren beziehungsweise Schütteln bei dieser Temperatur portionsweise während 10 Minuten 2,4 g (15 Millimol) Carbonyldiimidazol zugegeben. Dann wurde dem Gemisch ebenfalls bei 0 °C während 15 Minuten eine auf 0 °C gekühlte Lösung von 1,8 g (10 Millimol) L-Milchsäurebenzylester in 10 $cm^3$ wasserfreiem Tetrahydrofuran zugetropft. Das Reaktionsgemisch wurde dann 30 Minuten lang bei 0 °C und anschliessend 2 Stunden lang bei 20 °C gerührt beziehungsweise geschüttelt und über Nacht in einem Kühlschrank stehengelassen. Am nächsten Tag wurde das Lösungsmittel abdestilliert, der Rückstand in 30 $cm^3$ Chloroform gelöst und die Lösung 4-mal mit je 10 $cm^3$ einer n Salzsäure, anschliessend mit Wasser, dann mit je 15 $cm^3$ einer wässrigen Natriumbicarbonatlösung und schliesslich wieder mit Wasser ausgeschüttelt. Die organische Phase wurde über wasserfreiem Natriumsulfat getrocknet und bis zur Erreichung der Gewichtskonstanz eingedampft. Das als Rückstand verbliebene Öl wurde in einem Exsikkator über Phosphorpentoxyd getrocknet. So wurden 3,0 g (80% der Theorie) chromatographisch einheitlicher [N-(tert.-Butyloxycarbonyl)]-L-prolyl-L-milchsäurebenzylester mit einem $R_f^1$-Wert von 0,32 und einem $R_f^5$-Wert von 0,57 erhalten.

2. Stufe

[N-(Benzyloxycarbonyl)]-L-sarkosyl-[N-(nitro)]-L-arginyl- L-valyl- [O-(benzyl)]- L-tyrosyl- L-isoleucyl- [N-(dinitrophenyl)]- L-histidyl- L-prolyl-L-milchsäurebenzylester

Es wurden 2,85 g (7,5 Millimol) wie in der vorstehenden 1. Stufe beschrieben erhaltener [N-(tert.-Butyloxycarbonyl)]-L-prolyl-L -milchsäurebenzylester in 15 $cm^3$ einer 8 n Lösung von Chlorwasserstoff in Dioxan gelöst und die Lösung wurde 15 Minuten stehengelassen, dann mit 30 $cm^3$ wasserfreiem Äther versetzt und zur Trockne eingedampft. Das als Rückstand verbliebene freie Peptidesterhydrochlorid L-Prolyl-L-milchsäurebenzylesterhydrochlorid ($R_f^5$-Wert = 0,37) wurde in 20 $cm^3$ Dimethylformamid gelöst, der pH-Wert der Lösung wurde mit Triäthylamin auf 8 eingestellt und es wurden 2,9 g (5 Millimol) [N-(tert.-Butyloxycarbonyl)- N'-(dinitrophenyl)]- L-histidinpentafluorphenylester zugesetzt. Das Gemisch wurde 1 Stunde lang bei Raumtemperatur gerührt beziehungsweise geschüttelt, wobei der pH-Wert des Gemisches ständig auf 8 gehalten wurde. Dann wurde das Lösungsmittel abdestilliert, der Rückstand in Äthylacetat gelöst und die Lösung mit einer 10%-igen Citronensäurelösung, mit einer n Salzsäure, mit einer 5%-igen wässrigen Natriumbicarbonatlösung und schliesslich mit Wasser ausgeschüttelt. Die organische Phase wurde über wasserfreiem Natriumsulfat getrocknet und der nach dem Abdestillieren des Lösungsmittels als Rückstand verbliebene geschützte Peptidester [N- (tert.-Butyloxycarbonyl)- N'-(dinitrophenyl)]-L-histidyl-L-prolyl-L-milchsäurebenzylester ($R_f^2$-Wert = 0,77) wurde ohne Reinigung in 10 $cm^3$ einer 8 n Lösung von Chlorwasserstoff in Dioxan gelöst und nach 20 Minuten wurde das freie Peptidesterhydrochlorid [N-(Dinitrophenyl)]-L-histidyl-L-prolyl-L-milchsäurebenzylesterhydrochlorid durch Zugabe von wasserfreiem Äther gefällt, abfiltriert und mit Äther gewaschen. Das auf diese Weise erhaltene freie Peptidesterhydrochlorid [N-(Dinitrophenyl)]- L-histidyl- L-prolyl- L-milchsäurebenzylesterhydrochlorid ($R_f^4$-Wert = 0,10) wurde in 20 $cm^3$ Dimethylformamid gelöst, der pH-Wert der Lösung wurde mit Triäthylamin auf 8 eingestellt und es wurden 2,78 g (7,5 Millimol) [N-(tert.-Butyloxycarbonyl)]- L-isoleucinpentafluorphenylester zugesetzt. Das Reaktionsgemisch wurde unter Aufrechterhalten des pH-Wertes 1 Stunde gerührt beziehungsweise geschüttelt, dann wurde das Lösungsmittel abgedampft, der Rückstand in Äthylacetat gelöst und in der oben angegebenen Weise mit einer 10%-igen Citronensäurelösung, mit einer n Salzsäure, mit einer 5%-igen Natriumbicarbonatlösung und schliesslich mit Wasser ausgeschüttelt. Die organische Phase wurde dann über wasserfreiem Natriumsulfat getrocknet und eingedampft. Der Rückstand wurde mit n-Hexan verrieben und abfiltriert. Der auf diese Weise erhaltene geschützte Peptidester [N-(tert.-Butyloxycarbonyl)]- L-isoleucyl- [N-(dinitrophenyl)]- L-histidyl- L-prolyl-L-milchsäurebenzylester ($R_f^2$-Wert = 0,67) wurde in 10 $cm^3$ einer 8 n Lösung von Chlorwasserstoff in Dioxan gelöst und nach 15 Minuten wurde das freie Peptidesterhydrochlorid L-Isoleucyl-[N-(dinitrophenyl)]-L-histidyl-L-prolyl-L-milchsäurbenzylesterhydrochlorid ($R_f^5$-Wert = 0,52) durch Zugabe von wasserfreiem Äther gefällt. Dieses Produkt wurde in 20 $cm^3$ Dimethylformamid gelöst, der pH-Wert der Lösung wurde mit Triäthylamin auf 8 eingestellt und es wurden 3,24 g (6 Millimol) [N-(tert.-Butyloxycarbonyl)-O-(benzyl)]-L-tyrosinpentafluorphenylester zugesetzt. Das Gemisch wurde unter Aufrechterhalten des pH-Wertes 30 Minuten gerührt beziehungsweise geschüttelt, dann wurde das Lösungsmittel abgedampft, der Rückstand in Äthylacetat gelöst und die Lösung mit 0,22 $cm^3$ N,N-Dimethylaminoäthylamin versetzt. Nach 15 Minuten wurde die Lösung mit einer 10%-igen Citronensäurelösung, mit einer n Salzsäure, dann mit Wasser, mit einer 5%-igen Natriumbicarbonatlösung und schliesslich wieder mit Wasser ausgeschüttelt, über wasserfreiem Natriumsulfat getrocknet und eingedampft. Der Rückstand wurde mit einem Gemisch von n-Hexan und Äther im Volumverhältnis von 8 : 2 verrieben und abfiltriert. Der auf diese Weise erhaltene geschützte Peptidester [N-(tert.-Butyloxycarbonyl)-O-(benzyl) ] - L-tyrosyl- L-isoleucyl-[N-(dinitrophenyl)]- L-histidyl- L-prolyl- L-milchsäurebenzylester ($R_f^3$-Wert = 0,72) wurde in 10 $cm^3$ einer 8 n Lösung von Chlorwasserstoff in Dioxan gelöst und nach 15 Minuten wurde durch Zugabe von wasserfreiem Äther das freie Peptidesterhydrochlorid [O-(Benzyl)]-L-tyrosyl-L-isoleucyl-[N-(dinitrophenyl)]-L-histidyl-L-prolyl-L-milchsäurebenzylesterhydrochlorid gefällt. Dieses wurde in 20 $cm^3$ Dimethylformamid gelöst, der pH-Wert der Lösung wurde mit Triäthylamin auf 8 eingestellt und die Lösung wurde mit 2,6 g (6,8 Millimol) [N-(tert.-Butyloxycarbonyl)]-L-valinpentafluorphenylester versetzt. Das Gemisch wurde unter Aufrechterhalten des pH-Wertes 1 Stunde gerührt beziehungsweise geschüttelt und dann wurde das Lösungsmittel abgedampft, der Rückstand in Äthylacetat gelöst und die Lösung auf die oben angegebene Weise ausgeschüttelt. Die organische Phase wurde über wasserfreiem Natriumsulfat getrocknet und eingedampft und der Rückstand wurde mit wasserfreiem Äther verrieben und abfiltriert. Der erhaltene geschützte Peptidester [N-(tert.-Butyloxycarbonyl)]- L-valyl-[O-(benzyl)]- L-tyrosyl- L-isoleucyl-[N-(dinitrophenyl)] - L-histidyl- L-prolyl- L-milchsäurebenzylester ($R_f^3$-Wert = 0,83) wurde in 10 $cm^3$ einer 8 n Lösung von Chlorwasserstoff in Dioxan gelöst und nach 15 Minuten wurde durch Zugabe von wasserfreiem Äther das freie Peptidesterhydrochlorid L-Valyl-[O-(benzyl)]- L-tyrosil- L-isoleucyl-[N-(dinitrophenyl)]- L-histidyl- L-propyl- L-milchsäurebenzylesterhydrochlorid ($R_f^5$-Wert = 0,65) gefällt und abfiltriert. Dieses Produkt wurde in 20 $cm^3$ Dimethylformamid gelöst, der pH-Wert der Lösung wurde mit Triäthylamin auf 8 eingestellt und es wurden 2,2 g (5 Millimol) [N-(tert.-Butyloxycarbonyl)-N'-(nitro)]-L-argininpentafluorphenylester zugesetzt. Das Gemisch wurde unter Aufrechterhalten des pH-Wertes von 8 1 Stunde lang stehengelassen, dann mit 60 $cm^3$ Chloroform verdünnt und

zuerst mit einer 10%-igen Citronensäurelösung, dann mit einer n Salzsäure und schliesslich mit Wasser ausgeschüttelt. Die abgetrennte organische Phase wurde über wasserfreiem Natriumsulfat getrocknet und eingedampft. Der als Rückstand verbliebene geschützte Peptidester [N-(tert.-Butyloxycarbonyl)-N'-(nitro)]-L-arginyl-L-valyl-[O-(benzyl)]- L-tyrosyl- L-isoleucyl-[N-(dinitrophenyl)]- L-histidyl- L-prolyl- L-milchsäurebenzylester wurde mit einem Gemisch von Äthanol und Äther im Volumverhältnis von 1 : 1 verrieben und abfiltriert. Dieser geschützte Peptidester [N-(tert.-Butyloxy-carbonyl)-N'-(nitrol)]- L-arginyl- L-valyl-[O-(benzyl)]- L-tyrosyl-L- isoleucyl-[N-(dinitrophenyl)]- -histidyl- L-propyl- L-milchsäurebenzylester ($R_f^3$-Wert = 0,65) wurde in 10 $cm^3$ einer 8 n Lösung von Chlorwasserstoff in Dioxan gelöst und nach 15 Minuten wurde das freie Peptidesterhydrochlorid [N-(Nitro)]-L-arginyl-L-valyl-[O-(benzyl)]-L-tyrosyl- L-isoleucyl- [N-(dinitrophenyl)]-L-histidyl-L-prolyl-L-milchsäurebenzylesterhydrochlorid ($R_f^5$-Wert = 0,40) durch Zugabe von wasserfreiem Äther gefällt, abfiltriert, mit Äther gewaschen und getrocknet. Dieses Produkt wurde dann in 15 $cm^3$ Dimethylformamid gelöst, der pH-Wert der Lösung wurde mit Triäthylamin auf 8 eingestellt und es wurden 1,8 g (5 Millimol) [N-(Benzyloxycarbonyl)]-L-sarkosinpentafluorphenylester zugesetzt. Das Gemisch wurde unter Aufrechterhalten des pH-Wertes 30 Minuten gerührt beziehungsweise geschüttelt, dann mit 45 $cm^3$ Chloroform verdünnt und mit Wasser ausgeschüttelt. Die abgetrennte organische Phase wurde über wasserfreiem Natriumsulfat getrocknet und eingedampft, der als Rückstand verbliebene geschützte Peptidester [N-(Benzyloxycarbonyl)]-L-sarkosyl-[N-(nitro)]-L-arginyl-L-valyl-[O-(benzyl)]- L-tyrosyl-L-isoleucyl- [N-(dinitrophenyl)]- L-histidyl- L-prolyl-L-milchsäurebenzylester wurde mit wasserfreiem Äther verrieben und abfiltriert. So wurden 2,12 g [N-(Benzyloxycarbonyl)]-L-sarkosyl-[N-(nitro)]- L-arginyl-L-valyl-[O-(benzyl)]-L-tyrosyl- L-isoleucyl-[N-(dinitrophenyl)]- L-histidyl- L-prolyl-L-milchsäurebenzylester ($R_f^3$-Wert = 0,465 und Schmelzpunkt = 204 bis 213 °C) erhalten. (Diese Menge stellt eine Ausbeute von 29,6% der Theorie, bezogen auf L-Histidin, dar, woraus sich eine durchschnittliche Ausbeute von 82% der Theorie je Schritt ergibt).

3. Stufe

L-Sarkosyl-L-arginyl-L-valyl-L-tyrosyl-L-isoleucyl-L-histidyl-L-prolyl-L-milchsäure
(Entfernen der Schutzgruppen)

Es wurden 1,45 g (1 Millimol) des wie in der vorstehenden 2. Stufe beschrieben erhaltenen [N-(Benzyloxycarbonyl)]-L-sarkosyl-[N-(nitro)]-L-arginyl-L-valyl-[O-(benzyl)]- L-tyrosyl-L-isoleucyl-[N-(dinitrophenyl)]-L-histidyl-L-prolyl-L- milchsäurebenzylesters in 5 $cm^3$ Dimethylformamid gelöst und mit 2,3 $cm^3$ (30 Millimol) 2-Mercaptoäthanol versetzt. Das Gemisch wurde 1 Stunde gerührt beziehungsweise geschüttelt und dann mit wasserfreiem Äther versetzt und der auf diese Weise gefällte geschützte, aber keine Dinitrophenylgruppe mehr enthaltende Peptidester [N-(benzyloxycarbonyl)]-L-sarkosyl-[N-(nitro)]-L-arginyl-L-valyl-[O-(benzyl)]-L-tyrosyl-L-isoleucyl-L-histidyl-L-prolyl-L-milchsäurebenzylester wurde in Methanol gelöst und durch Zugabe von Äther wieder gefällt. Ausbeute: 1,14 g (89% der Theorie); $R_f^4$-Wert = 0,60.

Dieses Produkt wurde in 40 $cm^3$ eines Gemisches von Methanol, Essigsäure und Wasser im Volumverhältnis von 5 : 1 : 2 gelöst und mit 0,6 g von 10 Gew.-% Palladium auf Akitvkohle versetzt und unter kräftigem Rühren beziehungsweise Schütteln wurde 20 Stunden lang Wasserstoffgas durch das Gemisch geleitet. Dann wurde der Katalysator abfiltriert und mit demselben Lösungsmittelgemisch gewaschen und das Filtrat wurde zur Trockne eingedampft. Der Rückstand wurde in wasserfreiem Äthanol verrieben und abfiltriert. So wurde 0,7 g (84% der Theorie) L-Sarkosyl-L-arginyl- L-valyl- L-tyrosyl- L-isoleucyl-L-histidyl-L-prolyl-L-milchsäure erhalten.

4. Stufe

Reines L-Sarkosyl-L-arginyl-L-valyl-L-tyrosyl-L-isoleucyl-L-histidyl-L-prolyl-L-milchsäure

Das in der vorstehenden 3. Stufe erhaltene rohe Peptid L-Sarkosyl-L-arginyl-L-valyl-L-tyrosyl-L-isoleucyl- L-histidyl- L-prolyl- L-milchsäure wurde nach der vor den Beispielen beschriebenen allgemeinen Verfahrensweise gereinigt. Die $R_f$-Werte des erhaltenen gereinigten Octapeptides L-Sarkosyl-L-arginyl-L-valyl- L-tyrosyl- L-isoleucyl- L-histidyl-L-prolyl-L-milchsäure waren wie folgt:

$R_f^6$-Wert = 0,17

$R_f^7$-Wert = 0,40

$R_f^8$-Wert = 0,21.

Aminosäureanalyse:

| | |
|---|---|
| L-Prolin | = 1,0 [1], |
| L-Valin | = 1,0 [1], |
| L-Isoleucin | = 1,03 [1], |
| L-Tyrosin | = 0,85 [1], |
| L-Histidin | = 0,85 [1], |
| L-Arginin | = 0,96 [1] |
| und | |
| L-Sarkosin | = 1,0 [1]. |

Beispiel 2

L-Sarkosyl-L-arginyl-L-valyl-L-tyrosyl-L-isoleucyl-L-histidyl-L-prolyl-L-milchsäureäthylester {[Sarcosin[1], L-Milchsäureäthylester[8]]-Angiotensin-II}

1. Stufe

[N-(tert.-Butyloxycarbonyl)]-L-prolyl-L-milchsäureäthylester

Es wurden 4,3 g (20 Millimol) [N-(tert.-Butyloxycarbonyl)]-L-prolin in 20 $cm^3$ wasserfreiem Tetrahydrofuran gelöst und die Lösung wurde auf 0 °C abgekühlt und während 10 Minuten in kleinen Portionen mit 4,8 g (30 Millimol) Carbonyldiimidazol versetzt. Dann wurde dem Gemisch bei derselben Temperatur eine ebenfalls auf 0 °C abgekühlte

Lösung von 2,1 g (20 Millimol) L-Milchsäureäthylester in 20 $cm^3$ Tetrahydrofuran zugetropft. Das Reaktionsgemisch wurde 30 Minuten lang bei 0 °C und anschliessend 2 Stunden lang bei 20 °C gerührt beziehungsweise geschüttelt und über Nacht stehengelassen. Am nächsten Tag wurde das Tetrahydrofuran abdestilliert, der Rückstand in 40 $cm^3$ Äthylacetat gelöst und die Lösung 2-mal mit je 15 $cm^3$ einer n Salzsäure 1-mal mit Wasser, 2-mal mit je 20 $cm^3$ einer 5%-igen Natriumbicarbonatlösung und 1-mal mit Wasser in der angegebenen Reihenfolge ausgeschüttelt. Die abgetrennte organische Phase wurde über wasserfreiem Natriumsulfat getrocknet und eingedampft. Das als Rückstand verbliebene Öl wurde in einem Exsikkator bis zur Erreichung der Gewichtskonstanz getrocknet. So wurden 2,22 g (36% der Theorie) [N-(tert.-Butyloxycarbonyl)]-L-prolyl-L-milchsäureäthylester mit einem $R_f^3$-Wert von 0,77 und einem $R_f^1$-Wert von 0,36 erhalten.

2. Stufe

[N-(Benzyloxycarbonyl)]-L-sarkosyl- [N-(nitro)]-L-arginyl-L-valyl-[ O-(benzyl)]-L-tyrosyl-L-isoleucyl-[N-(dinitrophenyl)] -L-histidyl- L-prolyl-L-milchsäureäthylester

Es wurden 1,8 g (6 Millimol) wie in der vorstehenden 1. Stufe beschrieben erhaltener [N-(tert.-Butyloxycarbonyl)]- L-prolyl- L-milchsäureäthylester in 8 $cm^3$ einer 7,5 n Lösung von Chlorwasserstoff in Dioxan gelöst, die Lösung wurde 15 Minuten bei Raumtemperatur stehengelassen, dann wurden 30 $cm^3$ Äther zugegeben und das Gemisch wurde zur Trockne eingedampft. Das als Rückstand verbliebene freie Peptidesterhydrochlorid L-Prolyl-L-milchsäureäthylesterhydrochlorid-($R_f^4$-Wert = 0,31) wurde in 15 $cm^3$ Dimethylformamid gelöst, der pH-Wert der Lösung wurde mit Triäthylamin auf 8 eingestellt und zur Lösung wurden 2,9 g (5 Millimol) [N-(tert.-Butyloxycarbonyl)-N'-(dinitrophenyl)] -L-histidinpentafluorphenylester zugegeben. Das Gemisch wurde 1 Stunde lang bei Raumtemperatur stehengelassen, dann wurde das Lösungsmittel abgedampft, der Rückstand wurde in Äthylacetat gelöst, die Lösung wurde mit einer 10%-igen Citronensäurelösung, mit einer n Salzsäure, mit einer 5%-igen Natriumbicarbonatlösung und mit Wasser in der angegebenen Reihenfolge ausgeschüttelt und die abgetrennte organische Phase wurde über wasserfreiem Natriumsulfat getrocknet und eingedampft. Der als Rückstand verbliebene geschützte Peptidester [N-(tert.-Butyloxycarbonyl)-N'-(dinitrophenyl)]- L-histidyl- L-prolyl- L-milchsäureäthylester ($R_f^2$-Wert = 0,69) wurde sofort in 20 $cm^3$ einer 7,5 n Lösung von Chlorwasserstoff in Dioxan gelöst. Nach 20 Minuten wurde wasserfreier Äther zugesetzt. Das auf diese Weise gefällte freie Peptidesterhydrochlorid [[N-(Dinitrophenyl)] -L-histidyl-L-prolyl- L-milchsäureäthylesterhydrochlorid ($R_f^4$-Wert = 0,19) wurde in 15 $cm^3$ Dimethylformamid gelöst, der pH-Wert der Lösung wurde mit Triäthylamin auf 8 eingestellt und es wurden 3,0 g (7,5 Millimol) [N-(tert.-Butyloxycarbonyl)] -L-isoleucinpentafluorphenylester zugegeben. Die Lösung wurde unter Aufrechterhalten des pH-Wertes 30 Minuten stehengelassen, dann wurde das Lösungsmittel abgedampft, der Rückstand in Äthylacetat gelöst und die Lösung in der oben angegebenen Weise ausgeschüttelt. Die abgetrennte organische Phase wurde über wasserfreiem Natriumsulfat getrocknet und eingedampft und der Rückstand wurde mit einem Gemisch von Äther und n-Hexan im Volumverhältnis von 2 : 8 verrieben und abfiltriert. Der auf diese Weise erhaltene geschützte Peptidester [ N-(tert.-Butyloxycarbonyl)] -L-isoleucyl- [N-(dinitrophenyl)] -L-histidyl- L-prolyl-L -milchsäureäthylester ($R_f^3$-Wert = 0,36) wurde in 10 $cm^3$ einer 8 n Lösung von Chlorwasserstoff in Dioxan gelöst und nach 15 Minuten mit wasserfreiem Äther versetzt. Das gefällte freie Peptidesterhydrochlorid L-Isoleucyl- [N-(dinitrophenyl)] -L-histidyl- L-propyl- L-milchsäureäthylesterhydrochlorid ($R_f^5$-Wert = 0,27) wurde sofort in 20 $cm^3$ Dimethylformamid gelöst, der pH-Wert der Lösung wurde mit Triäthylamin auf 8 eingestellt und es wurden 3,24 g (6 Millimol) [N-(tert.-Butyloxycarbonyl)-O-(benzyl)]- L-tyrosinpentafluorphenylester zugesetzt. Das Gemisch wurde unter Aufrechterhalten des pH-Wertes 30 Minuten stehengelassen, dann wurde das Lösungsmittel abgedampft und der Rückstand in Äthylacetat gelöst. Die Lösung wurde mit 0,22 $cm^3$ N,N-Dimethylaminoäthylamin versetzt; nach 10 Minuten wurde die Lösung in der üblichen Weise ausgeschüttelt, getrocknet und eingedampft. Der Rückstand wurde mit n-Hexan verrieben und abfiltriert. Der auf diese Weise erhaltene geschützte Peptidester [N- (tert.-Butyloxycarbonyl) -O-(benzyl)] -L-tyrosyl-L-isoleucyl- [ N- (dinitrophenyl) ] -L-histidyl-L-prolyl-L- milchsäureäthylester ($R_f^2$-Wert = 0,61) wurde in 10 $cm^3$ einer 8 n Lösung von Chlorwasserstoff in Dioxan gelöst und nach 15 Minuten wurde das Produkt durch Zugabe von wasserfreiem Äther gefällt. Das erhaltene freie Peptidesterhydrochlorid [O-(Benzyl)-L-tyrosyl-L-isoleucyl- [N-(dinitrophenyl) ] -L-histidyl-L-prolyl-L-milchsäureäthylesterhydrochlorid ($R_f^2$-Wert = 0,33) wurde in 15 $cm^3$ Dimethylformamid gelöst, der pH-Wert der Lösung wurde mit Triäthylamin auf 8 eingestellt und es wurden 2,1 g (5,5 Millimol) [N-(tert.-Butyloxycarbonyl) ]-L-valinpentafluorphenylester zugesetzt. Das Gemisch wurde unter Aufrechterhalten des pH-Wertes 30 Minuten stehengelassen, dann wurde es eingedampft, der Rückstand wurde in Äthylacetat gelöst und die Lösung wurde auf die oben angegebene Weise ausgeschüttelt, getrocknet und eingedampft. Der Rückstand wurde mit n-Hexan verrieben und abfiltriert. Der auf diese Weise erhaltene geschützte Peptidester [N-(tert. -Butyloxycarbonyl) ]-L-valyl-[O-(benzyl) ]-L-tyrosyl-L-isoleucyl- [N-(dinitrophenyl) ]-L-histidyl-L-prolyl-L-milchsäureäthylester ($R_f^4$-Wert = 0,76) wurde sofort in 10 $cm^3$ einer 8 n Lösung von Chlorwasserstoff in Dimethylformamid gelöst und nach 15 Minuten wurde das Produkt durch Zugabe von wasserfreiem Äther gefällt. Das erhaltene freie Peptidesterhydrochlorid L-Valyl-[ – (benzyl)]-L-tyrosyl-L-isoleucyl- [N-(dinitrophenyl)] -L-histidyl-L- prolyl-L- milchsäureäthylesterhydrochlorid ($R_f^4$-Wert = 0,34) wurde in 20 $cm^3$ Dimethylformamid gelöst, der pH-Wert der Lösung wurde mit Triäthylamin auf 8 eingestellt und dann wurden 3,96 g (6 Millimol) [N-(tert.-Butyloxycarbonyl)- N'-(nitro)]- L-argininpentafluorphenylester zugesetzt. Das Gemisch wurde unter Aufrechterhalten des pH-Wertes 1 Stunde stehengelassen, dann mit 60 $cm^3$ Chloroform verdünnt, auf die oben angegebene Weise ausgeschüttelt, getrocknet und eingedampft. Der Rückstand wurde mit wasserfreiem Äthanol verrieben und abfiltriert. Der auf diese Weise erhaltene geschützte Peptidester [N-(tert.-Butyloxycarbonyl)- N'-(nitro)]-L-arginyl-L-valyl-[O-(benzyl)]- L-tyrosyl-L-isoleucyl-[N-(dinitrophenyl)]- L-histidyl- L-prolyl- L-milchsäureäthylester ($R_f^3$-Wert = 0,78) wurde in 10 $cm^3$ einer 8 n Lösung von Chlorwasserstoff in Dioxan gelöst und nach 15 Minuten wurde das Produkt durch Zugabe von wasserfreiem Äther gefällt. Das erhaltene freie Peptidesterhydrochlorid [N-(Nitro)] -L-arginyl-L-valyl- [O-(benzyl)] -L-tyrosyl-L-isoleucyl- [N-(dinitrophenyl)] -L-histidyl- L-prolyl-L- milchsäureäthylesterhydrochlorid ($R_f^4$-Wert = 0,56) wurde sofort in 20 $cm^3$ Dimethylformamid gelöst, der pH-Wert der Lösung wurde mit Triäthylamin auf 8 eingestellt und es wurden 2,12 g (5,5 Millimol) [N-(Benzyloxycarbonyl)] -L-sarkosinpentafluorphenylester zugesetzt. Das Gemisch wurde unter Aufrechterhalten des pH-Wertes 30 Minuten stehengelassen, dann mit 60 $cm^3$ Chloroform verdünnt, auf die oben angegebene Weise ausgeschüttelt, getrocknet und eingedampft, mit Äther verrieben, abfiltriert und aus Äthanol umkristallisiert. So wurden 1,21 g [N-(Benzyloxycarbonyl)] -L-sarkosyl- [N-(nitro)] -L-arginyl-L-valyl- [O-(benzyl)] -L-tyrosyl-L-isoleucyl- [N-(dinitrophenyl)] -L-histidyl-L-prolyl-L-milchsäureäthylester (Schmelzpunkt = 208 bis 212 °C und $R_f^3$-Wert = 0,5) erhalten. (Diese Menge stellt eine Ausbeute von 17,5% der Theorie, bezogen auf L-Histidin, dar, woraus sich eine durchschnittliche Ausbeute von 75% der Theorie je Schritt ergibt).

3. Stufe

L-Sarkosyl- L-arginyl- L-valyl- L-tyrosyl-L-isoleucyl- L-histidyl-L-prolyl- L-milchsäureäthylester (Entfernen der Schutzgruppen)

Es wurden 1,21 g (0,88 Millimol) wie in der vorstehenden 2. Stufe beschrieben erhaltener [N-(Benzyloxycarbonyl)] -L-sarkosyl- [N-(nitro)] -L-arginyl-L-valyl- [O-(benzyl)] -L-tyrosyl-L-isoleucyl-[N-(dinitrophenyl)] -L-histidyl-L-prolyl-L-milchsäureäthylester in 5 $cm^3$ Dimethylformamid gelöst und mit 3 $cm^3$ 2-Mercaptoäthanol versetzt. Das Gemisch wurde 1 Stunde lang bei Raumtemperatur gerührt beziehungsweise geschüttelt, dann wurde das Produkt durch Zugabe von wasserfreiem Äther gefällt, abfiltriert, gewaschen, in Methanol gelöst und durch Zugabe von wasserfreiem Äther wieder gefällt. Es wurde auf diese Weise 0,94 g (89% der Theorie) des geschützten, aber keine Dinitrophenylschutzgruppe mehr enthaltenden Peptidesters [N-(Benzyloxycarbonyl)] -L-sarkosyl-[N-(nitro)] -L-arginyl-L-valyl- [O-(benzyl)] -L-tyrosyl-L-isoleucyl- L-histidyl- L-prolyl- L-milchsäureäthylester ($R_f^4$-Wert = 0,59) erhalten. Dieses Peptid wurde in 20 $cm^3$ eines Gemisches von Methanol, Essigsäure und Wasser im Volumverhältnis von 5 : 1 : 1 gelöst, die Lösung wurde mit 0,5 g von 10 Gew.-% Palladium auf Aktivkohle als Katalysator versetzt und es wurde 20 Stunden lang Wasserstoffgas durch die Lösung geleitet, wobei das Reaktionsgemisch ständig kräftig gerührt beziehungsweise geschüttelt wurde. Das Fortschreiten der Reaktion wurde durch Dünnschichtchromatographie überwacht. Nach dem Ende der Reaktion wurde das Reaktionsgemisch filtriert, der auf dem Filter gebliebene Katalysator mit demselben Lösungsmittelgemisch gewaschen und das mit der Waschflüssigkeit vereinigte Filtrat zur Trockne eingedampft. Der Rückstand wurde mit einem Gemisch von Äthanol und Äther verrieben und abfiltriert. So wurde 0,72 g (96% der Theorie) L-Sarkosyl- L-arginyl- L-valyl- L-tyrosyl- L-isoleucyl- L-histidyl- L-prolyl- L-milchsäureäthylester erhalten.

4. Stufe

Reiner L-Sarkosyl- L-arginyl-L-valyl- L-tyrosyl-L-isoleucyl- L-histidyl- L-prolyl- L-milchsäureäthylester

Der in der vorstehenden 3. Stufe erhaltene rohe freie Peptidester L-Sarkosyl-L-arginyl-L-valyl-L-tyrosyl-L-isoleucyl-L-histidyl-L-prolyl- L-milchsäureäthylester wurde nach der vor den Beispielen beschriebenen allgemeinen Verfahrensweise gereinigt. Der reine Peptidester L-Sarkosyl-L-arginyl-L-valyl- L-tyrosyl- L-isoleucyl- L-histidyl- L-prolyl-L-milchsäureäthylester hatte die folgenden charakteristischen Eigenschaften:

$R_f^6$-Wert = 0,24
$R_f^7$-Wert =0,53
$R_f^8$-Wert = 0,36.

Aminosäureanalyse:

| | |
|---|---|
| L-Prolin | = 1,02 [1], |
| L-Valin | = 0,97 [1], |
| L-Isoleucin | = 1,08 [1], |
| L-Tyrosin | = 0,91 [1], |
| L-Histidin | = 1,00 [1], |
| L-Arginin | = 1,07 [1] |
| und | |
| L-Sarkosin | = 1,0 [1]. |

Beispiel 3

L-Sarkosyl -L-arginyl-L-valyl- L-tyrosyl-L-isoleucyl- L-histidyl- L-prolyl- L-2-hydroxy- 3-methylvaleriansäure
{[Sarcosin[1], L-2-Hydroxy-3-methyl-valeriansäure[8]]-Angiotensin-II}

1. Stufe

[N-(tert.-Butyloxycarbonyl)] -L-prolyl- L-2-hydroxy-3-methylvaleriansäurebenzylester

Es wurden 2,7 g (12,5 Millimol) [N-(tert.-Butyloxycarbonyl)]-L-prolin in 15 $cm^3$ wasserfreiem Tetrahydrofuran gelöst, die Lösung wurde auf 0 °C abgekühlt und es wurden während 10 Minuten in kleinen Portionen 3,2 g (20 Millimol) Carbonyldiimidazol zugesetzt. Das Gemisch wurde bei derselben Temperatur mit einer ebenfalls auf 0 °C gekühlten Lösung von 2,8 g (12,5 Millimol) L-2-Hydroxy-3-methylvaleriansäurebenzylester in 10 $cm^3$ Tetrahydrofuran versetzt und dann 30 Minuten lang bei 0 °C und anschliessend 2 Stunden lang bei 20 °C gerührt beziehungsweise geschüttelt und über Nacht in einem Kühlschrank stehengelassen. Am nächsten Tag wurde das Tetrahydrofuran abdestilliert, der Rückstand in 40 $cm^3$ Äthylacetat gelöst und die Lösung 2-mal mit je 15 $cm^3$ einer n Salzsäure, 1-mal mit Wasser, dann 1-mal mit einer 5%-igen Natriumbicarbonatlösung und schliesslich wieder mit Wasser ausgeschüttelt, über wasserfreiem Natriumsulfat getrocknet und eingedampft. Das als Rückstand verbliebene Öl wurde bei 25 °C bis zur Erreichung der Gewichtskonstanz getrocknet. So wurden 3,19 g (65% der Theorie) [N-(tert.-Butyloxycarbonyl)] -L-propyl- L-2-hydroxy-3-methylvaleriansäurebenzylester mit einem $R_f^2$-Wert von 0,90 und einem $R_f^1$-Wert von 0,33 erhalten.

2. Stufe

[N-(Benzyloxycarbonyl)] -L-sarkosyl- [N-(nitro)] -L-arginyl-L-valyl- [O-(benzyl)] -L-tyrosyl-L-isoleucyl- [N-(dinitrophenyl)] -L-histidyl-L-prolyl-L-2-hydroxy-3-methylvaleriansäurebenzylester

Es wurden 2,52 g (6 Millimol) wie in der vorstehenden 1. Stufe beschrieben erhaltener [N-(tert.-Butyloxycarbonyl)] -L-propyl- L-2-hydroxy-3-methylvaleriansäurebenzylester in 8 $cm^3$ einer 8 n Lösung von Chlorwasserstoff in Dioxan gelöst und die Lösung wurde 15 Minuten lang bei Raumtemperatur stehengelassen, dann mit 30 $cm^3$ wasserfreiem Äther versetzt und zur Trockne eingedampft. Das als Rückstand verbliebene freie Peptidesterhydrochlorid L-Prolyl-L-2-hydroxy-3-methylvaleriansäurebenzylesterhydrochlorid ($R_f^4$-Wert = 0,30) wurde in 15 $cm^3$ Dimethylformamid gelöst, der pH-Wert der Lösung wurde mit Triäthylamin auf 8 eingestellt und es wurden 2,9 g (5 Millimol) [N-(tert.-Butyloxycarbonyl)-N' -(dinitrophenyl)] -L-histidinpentafluorphenylester zugesetzt. Das Gemisch wurde 1 Stunde lang bei konstant gehaltenem pH-Wert stehengelassen und dann eingedampft und der verbliebene Rückstand wurde in Äthylacetat gelöst. Die Lösung wurde mit einer n Salzsäure, mit Wasser und mit einer 5%-igen wässrigen Natriumbicarbonatlösung in der angegebenen Reihenfolge ausgeschüttelt, über wasserfreiem Natriumsulfat getrocknet und eingedampft. Der als Rückstand verbliebene geschützte Peptidester [N-(tert.-Butyloxycarbonyl)-N'-(dinitrophenyl)] -L-histidyl-L-prolyl- L-2-hydroxy-3-methylvaleriansäurebenzylester ($R_f^2$-Wert = 0,67) wurde in 10 $cm^3$ einer 8 n Lösung von Chlorwasserstoff in Dioxan gelöst und nach 20 Minuten wurde durch Zugabe von wasserfreiem Äther das freie Peptidesterhydrochlorid [N-(Dinitrophenyl)], L-histidyl- L-prolyl- L-2-hydroxy-3-methylvaleriansäurebenzylesterhydrochlorid ($R_f^4$-Wert = 0,18) gefällt.

Das auf die obige Weise erhaltene Peptidesterhydrochlorid [N-(Dinitrophenyl)] -L-histidyl-L-prolyl- L-2-hydroxy-3-methylvaleriansäurebenzylesterhydrochlorid wurde in 15 $cm^3$ Dimethylformamid gelöst, der pH-Wert der Lösung wurde mit Triäthylamin auf 8 eingestellt und es wurden 2,4 g (6 Millimol) [N-(tert.-Butyloxycarbonyl)] -L-isoleucinpentafluorphenylester zugesetzt. Das Gemisch wurde bei konstant gehaltenem pH-Wert 1 Stunde stehengelassen und dann eingedampft. Der Rückstand wurde in Chloroform gelöst und die Lösung wurde in üblicher Weise mit Wasser, mit einer n Salzsäure und mit einer 5%-igen wässrigen Natriumbicarbonatlösung ausgeschüttelt, über wasserfreiem Natriumsulfat getrocknet und eingedampft. Der Rückstand wurde mit n-Hexan und nach dem Abdekantieren des n-Hexanes mit wasserfreiem Äther verrieben und abfiltriert. Der auf diese Weise erhaltene geschützte Peptidester [N-(tert.-Butyloxycarbonyl)] -L-isoleucyl- [N-(dinitrophenyl)] -L-histidyl- L-prolyl-L-2-hydroxy-3-methylvaleriansäurebenzylester ($R_f^2$-Wert = 0,65) wurde in 10 $cm^3$ einer 8 n Lösung von Chlorwasserstoff in Dioxan gelöst und nach 15 Minuten langem Stehenlassen wurde durch Zugabe von wasserfreiem Äther das freie Peptidesterhydrochlorid L-Isoleucyl- [N-(dinitrophenyl)] -L-histidyl-L-prolyl-L-2-hydroxy-3- methylvaleriansäurebenzylesterhydrochlorid ($R_f^4$-Wert = 0,27) gefällt. Dieses Produkt wurde in 15 $cm^3$ Dimethylformamid gelöst, der pH-Wert der Lösung wurde mit Triäthylamin auf 8 eingestellt und es wurden 2,96 g [N-(tert.-Butyloxycarbonyl) -O- (benzyl)] -L-tyrosinpentafluorphenylester zugesetzt. Das Gemisch wurde bei konstant gehaltenem pH-Wert 30 Minuten stehengelassen und dann eingedampft. Der Rückstand wurde in Äthylacetat gelöst und mit 0,22 $cm^3$ N,N-Dimethylaminoäthylamin versetzt und die erhaltene Lösung wurde 10 Minuten stehengelassen. Dann wurde die Lösung mit einer 10%igen wässrigen Citronensäurelösung, mit einer n Salzsäure, mit einer 5%-igen wässrigen Natriumbicarbonatlösung und schliesslich mit Wasser ausgeschüttelt, über wasserfreiem Natriumsulfat getrocknet und eingedampft. Der Rückstand wurde mit n-Hexan verrieben und abfiltriert. Der auf diese Weise erhaltene geschützte Peptidester [N- (tert.-Butyloxycarbonyl) -O- (benzyl)] -L-tyrosyl- L-isoleucyl- [N-(dinitrophenyl)]- L-histidyl-L-prolyl- L-2-hydroxy-3 -methylvaleriansäurebenzylester ($R_f^3$-Wert = 0,75) wurde in 10 $cm^3$ einer 8 n Lösung von Chlorwasserstoff in Dioxan gelöst und nach 15 Minuten wurde durch Zugabe von wasserfreiem Äther das freie Peptidesterhydrochlorid [O-(Benzyl)] -L-tyrosyl-L-isoleucyl-[N-(dinitrophenyl)]-L-histidyl- L-prolyl-L -2-hydroxy-3-methylvaleriansäurebenzylesterhydrochlorid ($R_f^4$-Wert = 0,60) gefällt. Dieses wurde sofort in 20 $cm^3$ Dimethylformamid gelöst, der pH-Wert der Lösung wurde mit Triäthylamin auf 8 eingestellt und es wurden 2,6 g (6 Millimol) [N-(tert.-Butyloxycarbonyl)]-L-valinpentafluorphenylester zugesetzt. Die erhaltene Lösung wurde bei konstant gehaltenem pH-Wert 1 Stunde stehengelassen und dann eingedampft. Der Rückstand wurde in Chloroform gelöst, die Lösung wurde mit einer 10%-igen Citronensäure, mit einer n Salzsäure,

mit einer 5%-igen wässrigen Natriumbicarbonatlösung und schliesslich mit Wasser ausgeschüttelt, über wasserfreiem Natriumsulfat getrocknet und eingedampft. Der Rückstand wurde mit n-Hexan verrieben und abfiltriert. Der auf diese Weise erhaltene geschützte Peptidester [N-(tert.-Butyloxycarbonyl)]-L-valyl-[O-(benzyl)] -L-tyrosyl-L-isoleucyl- [N-(dinitrophenyl)] -L- histidyl-L- prolyl-L-2-hydroxy-3- methylvaleriansäurebenzylester ($R_f^3$-Wert = 0,71) wurde in 10 $cm^3$ einer 8 n Lösung von Chlorwasserstoff in Dioxan gelöst, die Lösung wurde bei Raumtemperatur 15 Minuten stehengelassen und dann wurde durch Zugabe von wasserfreiem Äther das freie Peptidesterhydrochlorid L-Valyl-[O-(benzyl)]-L-tyrosyl-L-isoleucyl- [N-(dinitrophenyl)] -L-histidyl- L-prolyl-L -2-hydroxy-3- methylvaleriansäurebenzylester ($R_f^4$-Wert = 0,34) gefällt. Dieses Produkt wurde sofort in 20 $cm^3$ Dimethylformamid gelöst, der pH-Wert der Lösung wurde mit Triäthylamin auf 8 eingestellt und es wurden 2,64 g (6 Millimol) [N-(tert. -Butyloxycarbonyl)-N'-(nitro)] -L-argininpentafluorphenylester zugesetzt. Die Lösung wurde unter Aufrechterhalten des pH-Wertes von 8 1 Stunde lang stehengelassen und dann eingedampft, der Rückstand wurde in Chloroform gelöst und diese Lösung wurde mit einer 10% Dimethylformamid enthaltenden 10%-igen wässrigen Citronensäurelösung, mit einer n Salzsäure, mit einer 5%-igen wässrigen Natriumbicarbonatlösung und mit Wasser in der angegebenen Reihenfolge ausgeschüttelt, über wasserfreiem Natriumsulfat getrocknet und eingedampft. Der Rückstand wurde mit einem Gemisch von Äther und Äthanol im Volumverhältnis von 8 : 2 verrieben und abfiltriert. Der auf diese Weise erhaltene geschützte Peptidester [N-(tert. -Butyloxycarbonyl)-N'-(nitro)] -L-arginyl-L-valyl- [O-(benzyl)] -L-tyrosyl-L-isoleucyl- [N-(dinitrophenyl)] -L-histidyl-L-prolyl-L-2-hydroxy-3 -methylvaleriansäurebenzylester ($R_f^3$-Wert = 0,63) wurde in 10 $cm^3$ einer 8 n Lösung von Chlorwasserstoff in Dioxan gelöst und 15 Minuten stehengelassen und dann wurde durch Zugabe von wasserfreiem Äther das freie Peptidesterhydrochlorid [N-(Nitro)]-L-arginyl-L-valyl- [O-(benzyl)] -L-tyrosyl-L-isoleucyl- [N-(dinitrophenyl)] - L-histidyl- L-prolyl-L-2- hydroxy-3- methylvaleriansäurebenzylesterhydrochlorid gefällt, abfiltriert, gewaschen und getrocknet. Das auf diese Weise erhaltene freie Peptidesterhydrochlorid [N-(Nitro)]-L-arginyl-L-valyl- [O-(benzyl)] -L-tyrosyl-L-isoleucyl-[N-(dinitrophenyl)] -L-histidyl- L-prolyl-L-2-hydroxy-3- methylvaleriansäurebenzylesterhydrochlorid ($R_f^5$-Wert = 0,45) wurde in 20 $cm^3$ Dimethylformamid gelöst, der pH-Wert der Lösung wurde mit Triäthylamin auf 8 eingestellt und es wurden 2,3 g (6 Millimol) [N-(Benzyloxycarbonyl)] -L-sarkosinpentafluorphenylester zugesetzt. Die Lösung wurde bei konstant gehaltenem pH-Wert 30 Minuten stehengelassen, dann wurde sie mit 60 $cm^3$ Chloroform verdünnt, mit einer n Salzsäure und anschliessend mit Wasser ausgeschüttelt, über wasserfreiem Natriumsulfat getrocknet und eingedampft. Der Rückstand wurde mit einem Gemisch von Äther und Äthanol im Volumverhältnis von 8 : 2 verrieben, abfiltriert und gewaschen. So wurden 2,36 g [N-(Benzyloxycarbonyl)] -L-sarkosyl- [N-(nitro)] -L-arginyl-L-valyl- [O-(benzyl)] -L-tyrosyl-L-isoleucyl- [N-(dinitrophenyl)] -L-histidyl-L-prolyl- L-2-hydroxy-3-methylvalerian-säurebenzylester mit einem Schmelzpunkt von 193 bis 202 >C, einem $R_f^3$-Wert von 0,30 und einem $R_f^4$-Wert von 0,86 erhalten. (Diese Menge stellt eine Ausbeute von 30% der Theorie, bezogen auf L-Histidin, dar, woraus sich eine durchschnittliche Ausbeute von 82% der Theorie je Schritt ergibt).

3. Stufe

L-Sarkosyl- L-arginyl- L-valyl- L-tyrosyl- L-isoleucyl- L-histidyl- L-prolyl- L-2-hydroxy-3-methylvaleriansäure

(Entfernen der Schutzgruppen)

Es wurden 2,0 g (1,25 Millimol) des wie in der vorstehenden 2. Stufe beschrieben erhaltenen [N-(Benzyloxycarbonyl)] -L-sarkosyl- [N-(nitro)] -L-arginyl- L-valyl- [O-(benzyl)] -L-tyrosyl- L-isoleucyl-[N-(dinitrophenyl)] -L-histidyl- L-prolyl- L-2-hydroxy-3-methylvaleriansäurebenzylesters in 5 $cm^3$ Dimethylformamid gelöst, die Lösung wurde mit 2,8 $cm^3$ 2-Mercaptoäthanol versetzt, 1 Stunde gerührt beziehungsweise geschüttelt und dann wurde das Produkt durch Zugabe von wasserfreiem Äther gefällt, mit Äther gewaschen, in Methanol gelöst und durch Zugabe von wasserfreiem Äther noch einmal gefällt. Es wurden auf diese Weise 1,44 g (80% der Theorie) des geschützten, aber keine Dinitrophenylgruppe mehr enthaltenden Peptidesters [N-(Benzylocycarbonyl)] -L-sarkosyl-[N-(nitro)] -L-arginyl- L-valyl- [O-(benzyl)] -L-tyrosyl- L-isoleucyl- L-histidyl- L-prolyl- L-2-hydroxy-3-methylvaleriansäurebenzylester ($R_f^4$-Wert = 0,41) erhalten.

Dieses Produkt wurde in einem Gemisch von Methanol, Essigsäure und Wasser im Volumverhältnis von 5 : 2 : 1 gelöst und mit 0,7 g von 10 Gew.-% Palladium auf Aktivkohle als Katalysator versetzt und durch das kräftig gerührte beziehungsweise geschüttelte Gemisch wurde 20 Stunden lang Wasserstoffgas geleitet. Nach dem Ende der Reaktion wurde der Katalysator abfiltriert und mit 20 $cm^3$ desselben Lösungsmittelgemisches gewaschen; das mit der Waschflüssigkeit vereinigte Filtrat wurde zur Trockne eingedampft. Der Rückstand wurde mit einem Gemisch von gleichen Volumteilen von Äthanol und Äther verrieben und abfiltriert. So wurde 0,65 g (67%) der Theorie) L-Sarkosyl- L-arginyl- L-valyl- L-tyrosyl-L-isoleucyl- L-histidyl- L-prolyl- L-2-hydroxy-3-methylvaleriansäure erhalten.

4. Stufe

Reines L-Sarkosyl- L-arginyl- L-valyl- L-tyrosyl-L-isoleucyl- L-histidyl- L-prolyl- L-2-hydroxy-3-methylvaleriansäure

Das in der vorstehenden 3. Stufe erhaltene rohe Peptid L-Sarkosyl- L-arginyl- L-valyl- L-tyrosyl-L-isoleucyl- L-histidyl- L-prolyl- L-2-hydroxy-3-methylvaleriansäure wurde nach der vor den Beispielen beschriebenen allgemeinen Verfahrensweise

gereinigt. Das reine Peptid L-Sarkosyl- L-arginyl- L-valyl- L-tyrosyl- L-isoleucyl- L-histidyl- L-prolyl- L-2-hydroxy-3-methylvaleriansäure hatte die folgenden charakteristischen Eigenschaften:

$R_f^6$-Wert = 0,26
$R_f^7$-Wert = 0,52
$R_f^8$ = 0,30.

Aminosäureanalyse:

| | |
|---|---|
| L-Rolin | = 1,06 [1], |
| L-Valin | = 1,03 [1], |
| L-Isoleucin | = 1,03 [1], |
| L-Tyrosin | = 0,65 [1], |
| L-Histidin | = 0,99 [1], |
| L-Arginin | = 0,95 [1], |
| und | |
| L-Sarkosin | = 1,0 [1]. |

Der als Ausgangsstoff verwendete L-2-Hydroxy-3-methylvaleriansäurebenzylester ist wie folgt beschrieben hergestellt worden.

Es wurden 7,12 g (40 Millimol) des Natriumsalzes von L-2-Hydroxy-3-methylvaleriansäure in 10 $cm^3$ Äthylacetat suspendiert und mit 20 $cm^3$ einer 4 n Lösung von Chlorwasserstoff in Äthylacetat versetzt. Das Gemisch wurde 2 Stunden lang bei Raumtemperatur gerührt beziehungsweise geschüttelt, dann wurde der pH-Wert des Gemisches mit Triäthylamin auf 8 eingestellt und der gefällte Niederschlag wurde abfiltriert und 2-mal mit je 20 $cm^3$ Äthylacetat gewaschen. Der pH-Wert des mit den Waschflüssigkeiten vereinigten Filtrates wurde mit Triäthylamin auf 6 eingestellt und dann wurden 8 $cm^3$ (60 Millimol) Benzylbromid und 8,4 $cm^3$ (60 Millimol) Triäthylamin zugesetzt. Das Gemisch wurde 8 Stunden lang unter Rückfluss zum Sieden erhitzt, das gefällte anorganische Salz wurde afbiltriert und das Filtrat wurde mit Wasser, mit einer n Salzsäure, mit einer 5%-igen wässrigen Natriumbicarbonatlösung und erneut mit Wasser in der angegebenen Reihenfolge ausgeschüttelt, über wasserfreiem Natriumsulfat getrocknet und eingedampft. So wurden 5,6 g (63% der Theorie) L-2-Hydroxy-3-methylvaleriansäurebenzylester erhalten. Dieses rohe Produkt wurde durch Vakuumdestillation gereinigt. Der reine L-2-Hydroxy-3-methylvaleriansäurebenzylester hatte einen Siedepunkt von 134 bis 136 °C/ 5 mm Hg und einen $[\alpha]\frac{25}{D}$ -Wert von − 13,9° (C = 1; in Aceton).

**Patentansprüche für Vertragsstaaten: BE, CH, LI, DE, FR, GB, IT, NL, SE,**

1. Peptide der allgemeinen Formel

X–Arg–Val–Tyr–Ile–His–Pro–Y–O–A I,

worin

X einen Acylrest einer N-Methylaminosäure oder einer in der α-Stellung eine Aminooxygruppe oder eine Hydroxygruppe aufweisenden aliphatischen Carbonsäure, jeweils in der L-Konfiguration im Falle eines asymmetrischen Kohlenstoffatomes,

Arg einen Rest von L-Arginin,
Val einen Rest von L-Valin,
Tyr einen Rest von L-Tyrosin,
Ile einen Rest von L-Isoleucin,
His einen Rest von L-Histidin,
Pro einen Rest von L-Prolin,
Y einen Rest einer aliphatischen α-Hydroxycarbonsäure mit 1 gegenüber dieser Säure fehlenden Wasserstoffatom ihrer Hydroxygruppe ausser dem Fehlen der Hydroxygruppe der Carboxylgruppe, und
A Wasserstoff oder einen Alkylrest mit 1 bis 5 Kohlenstoffatom(en) darstellen,
sowie ihre Säureadditionssalze und pharmazeutisch brauchbaren Komplexe.

2. Peptide nach Anspruch 1, dadurch gekennzeichnet, dass der Acylrest einer N-Methylaminosäure, für den X stehen kann, der Sarkosylrest ist.

3. Peptide nach Anspruch 1 oder 2, dadurch gekennzeichnet, dass der Rest einer aliphatischen α-Hydroxycarbonsäure, für den Y steht, ein solcher mit einem asymmetrischen Kohlenstoffatom in der L-Konfiguration, insbesondere von L-Milchsäure oder L-2-Hydroxy-3-methylvaleriansäure, ist.

4. Peptide nach Anspruch 1 bis 3, dadurch gekennzeichnet, dass der Alkylrest, für den A stehen kann, ein solcher mit 1 oder 2, ganz besonders 1, Kohlenstoffatom(en) ist.

5. L-Sarkosyl- L-arginyl- L-valyl- L-tyrosyl-L-isoleucyl- L-histidyl- L-prolyl- L-2-hydroxy-3-methylvaleriansäure und ihre Alkylester mit 1 bis 5 Kohlenstoffatomen im Alkylteil.

6. L-Sarkosyl- L-arginyl- L-valyl- L-tyrosyl-L-isoleucyl-L-histidyl-L-prolyl-L-milchsäure und ihre Alkylester mit 1 bis 5 Kohlenstoffatomen im Alkylteil.

7. Verfahren zur Herstellung der Verbindungen nach Anspruch 1 bis 6, dadurch gekennzeichnet, dass man in in der Peptidchemie an sich bekannter Weise eine passende aliphatische α-Hydroxycarbonsäure oder einen passenden Alkylester derselben mit 1 bis 5 Kohlenstoffatomen im Alkylteil oder einen sonstigen Ester derselben nur mit Schutzfunktion der Estergruppe mit der nachfolgend einzubauenden an ihrem endständigen Stickstoffatom eine abspaltbare Schutzgruppe und, soweit erforderlich, an einem gegebenenfalls vorliegenden anderen Stickstoffatom oder Sauerstoffatom eine weitere abspaltbare Schutzgruppe aufweisenden Aminosäure und/oder dem nachfolgend einzubauenden an seinem endstündigen Stickstoffatom eine abspaltbare Schutzgruppe und, soweit erforderlich, an einem oder mehreren gegebenenfalls vorliegenden anderen Stickstoff- und/oder Sauerstoffatom(en) eine beziehungsweise mehrere Schutzgruppe(n) aufweisenden Peptidfragment beziehungsweise einem Esterderivat desselben kondensiert und gegebenenfalls mit dem erhaltenen am endständigen Stickstoffatom geschützten Peptidzwischenprodukt und dem beziehungsweise den durch etwaige weitere derartige Kondensationen erhaltenen am endständigen Stickstoffatom geschützten weiteren Peptidzwischenprodukt(en) nach in an sich bekannter Weise erfolgendem Entfernen der Schutzgruppe des endständigen Stickstoffatomes eine weitere beziehungsweise weitere Kondensation(en) mit beziehungsweise jeweils mit der nachfolgend einzubauenden an ihrem endständigen Stickstoffatom eine absp altbare Schutzgruppe und, soweit erforderlich, an einem gegebenenfalls vorliegenden anderen Stickstoffatom oder Sauerstoffatom eine weitere abspaltbare Schutzgruppe aufweisenden Aminosäure und/oder dem nachfolgend einzubauenden an seinem endständigen Stickstoffatom eine abspaltbare Schutzgruppe und, soweit erforderlich, an einem oder mehreren gegebenenfalls vorliegenden anderen Stickstoff- und/oder Sauerstoffatom(en) eine beziehungsweise mehrere Schutzgruppe(n) aufweisenden Peptidfragment beziehungsweise einem Esterderivat desselben vornimmt, wobei man so viele Kondensationen durchführt, wie es zum Einbau aller gewünschten Aminosäureeinheiten erforderlich ist, sowie danach in an sich bekannter Weise vom erhaltenen geschützten Peptidderivat die Schutzgruppe des endständigen Stickstoffatomes und die etwaige(n) Schutzgruppe(n) von anderen Stickstoffatomen beziehungsweise Sauerstoffatomen sowie gegebenenfalls die Aminogruppe der gegebenenfalls vorliegenden Aminooxygruppe selektiv schrittweise oder in einem Schritt entfernt, worauf man gegebenenfalls in an sich bekannter Weise das erhaltene Peptid der allgemeinen Formel I in ein Säureadditionssalz oder einen Komplex desselben überführt beziehungsweise gegebenenfalls das erhaltene Säureadditionssalz des Peptides der allgemeinen Formel I in ein anderes Säureadditionssalz oder in das Peptid der allgemeinen Formel I überführt.

8. Verfahren nach Anspruch 7, dadurch gekennzeichnet, dass man

a) zur Herstellung der Peptide der allgemeinen Formel I, bei welchen A für Wasserstoff steht, durch die Kondensation(en) als geschützte Peptide solche der allgemeinen Formel

B–X–Arg[C]–Val–Tyr[D]–Ile–His[E]–Pro–Y–O–F

II,

worin

B für eine durch Acidolyse oder katalytische Hydrogenolyse entfernbare Schutzgruppe, insbesondere eine Benzyloxycarbonyl- oder tert.-Butyloxycarbonylgruppe, steht,

C eine Schutzgruppe zum zeitweiligen Schutz der Guanidinogruppe des Restes von Arginin, insbesondere eine Nitro- oder p-Toluolsulfonylgruppe, bedeutet,

D eine Schutzgruppe zum zeitweiligen Schutz der aromatischen Hydroxygruppe des Restes von Tyrosin, insbesondere eine, gegebenenfalls substituierte, Benzylgruppe, darstellt,

E für eine Schutzgruppe zum zeitweiligen Schutz der Imidazolgruppe des Restes von Histidin, insbesondere eine Dinitrophenylgruppe, steht,

F eine gegen die Einwirkung von milden Säuren widerstandsfähige, aber durch katalytische Hydrogenolyse oder durch die Einwirkung von stärkeren Säuren entfernbare Schutzgruppe zum Schutz der Carboxylgruppe des Restes der C-endständigen α-Hydroxycarbonsäure bedeutet und

X, Arg, Val, Tyr, Ile, His, Pro, Y und A wie in den Ansprüchen 1 bis 4 festgelegt sind, aufbaut oder

b) zur Herstellung der Peptide der allgemeinen Formel I, bei welchen A einen Alkylrest mit 1 bis 5 Kohlenstoffatomen bedeutet, durch die Kondensation(en) als geschützte Peptide solche der allgemeinen Formel

B–X–Arg[C]–Val–Tyr[D]–Ile– His[E]–Pro–Y–O–A

III,

worin

B, C, D und E wie vorstehend festgelegt sind und

X, Arg, Val, Tyr, Ile, His, Pro, Y und A wie in den Ansprüchen 1 bis 4 festgelegt sind, aufbaut.

9. Verfahren nach Anspruch 7 oder 8 zur Herstellung von Peptiden der allgemeinen Formel I, bei welchen X für einen Acylrest einer in der α-Stellung eine Aminooxygruppe aufweisenden aliphatischen Carbonsäure steht, dadurch gekennzeichnet, dass man durch die Kondensation(en) die entsprechenden geschützten Peptide der allgemeinen Formeln II beziehungsweise III, bei welchen X ebenfalls für einen Acylrest einer in der α-Stellung eine Aminooxygruppe aufweisenden aliphatischen Carbonsäure steht, aufbaut und das Abspalten der Schutzgruppe, für welche B steht, sowie gegebenenfalls der anderen durch Acidolyse abspaltbaren Schutzgruppen von ihnen durch Acidolyse, insbesondere durch Behandeln mit Fluorwasserstoffsäure, durchführt.

10. Verfahren nach Anspruch 7 oder 8 zur Herstellung von Peptiden der allgemeinen Formel I, bei welchen X für einen Acylrest einer in der α-Stellung eine Hydroxygruppe aufweisenden aliphatischen Carbonsäure steht, dadurch gekennzeichnet, dass man durch die Kondensation(en) die entsprechenden Peptide der allgemeinen Formeln II beziehungsweise III, bei welchen jedoch X für einen Acylrest einer in der α-Stellung eine Aminooxygruppe aufweisenden aliphatischen Carbonsäure steht, aufbaut und das Abspalten der Schutzgruppe, für welche B steht, sowie gegebenenfalls der anderen durch katalytische Hydrogenolyse abspaltbaren Schutzgruppen von ihnen durch katalytische Hydrierung unter gleichzeitiger Abspaltung der Aminogruppe der genannten α-Aminooxygruppe durchführt.

11. Arzneimittel beziehungsweise Diagnostica, gekennzeichnet durch einen Gehalt an 1 oder mehr Verbindungen nach Anspruch 1 bis 6 als Wirkstoff beziehungsweise Wirkstoffen, zweckmässigerweise zusammen mit üblichen pharmazeutischen Konfektionierungsmitteln.

**Patentansprüche für Vertragsstaat AT**

1. Verfahren zur Herstellung von Peptiden der allgemeinen Formel

X–Arg–Val–Tyr–Ile–His–Pro–Y–O–A I,

worin

X einen Acylrest einer N-Methylaminosäure oder einer in der α-Stellung eine Aminooxygruppe oder eine Hydroxygruppe aufweisenden aliphatischen Carbonsäure, jeweils in der L-Konfiguration im Falle eines asymmetrischen Kohlenstoffatomes,

Arg einen Rest von L-Arginin,

Val einen Rest von L-Valin,

Tyr einen Rest von L-Tyrosin,

Ile einen Rest von L-Isoleucin,

His einen Rest von L-Histidin,

Pro einen Rest von L-Prolin,

Y einen Rest einer aliphatischen α-Hydroxycarbonsäure mit 1 gegenüber dieser Säure fehlenden Wasserstoffatom ihrer Hydroxygruppe ausser dem Fehlen der Hydroxygruppe der Carboxylgruppe, und

A Wasserstoff oder einen Alkylrest mit 1 bis 5 Kohlenstoffatom(en) darstellen,

sowie ihren Säureadditionssalzen und pharmazeutisch brauchbaren Komplexen, dadurch gekennzeichnet, dass man in in der Peptidchemie an sich bekannter Weise eine passende aliphatische α-Hydroxycarbonsäure oder einen passenden Alkylester derselben mit 1 bis 5 Kohlenstoffatomen im Alkylteil oder einen sonstigen Ester derselben nur mit Schutzfunktion der Estergruppe mit der nachfolgend einzubauenden an ihrem endständigen Stickstoffatom eine abspaltbare Schutzgruppe und, soweit erforderlich, an einem gegebenenfalls vorliegenden anderen Stickstoffatom oder Sauerstoffatom eine weitere abspaltbare Schutzgruppe aufweisenden Aminosäure und/oder dem nachfolgend einzubauenden an seinem endständigen Stickstoffatom eine abspaltbare Schutzgruppe und, soweit erforderlich, an einem oder mehreren gegebenenfalls vorliegenden anderen Stickstoff- und/oder Sauerstoffatom(en) eine beziehungsweise mehrere Schutzgruppe(n) aufweisenden Peptidfragment beziehungsweise einem Esterderivat desselben kondensiert und gegebenenfalls mit dem erhaltenen am enständigen Stickstoffatom geschützten Peptidzwischenprodukt und dem beziehungsweise den durch etwaige weitere derartige Kondensationen erhaltenen am endständigen Stickstoffatom geschützten weiteren Peptidzwischenprodukt(en) nach in an sich bekannter Weise erfolgendem Entfernen der Schutzgruppe des endständigen Stickstoffatomes eine weitere beziehungsweise weitere Kondensation(en) mit beziehungsweise jeweils mit der nachfolgend einzubauenden an ihrem endständigen Stickstoffatom eine abspaltbare Schutzgruppe und, soweit erforderlich, an einem gegebenenfalls vorliegenden anderen Stickstoffatom oder Sauerstoffatom eine weitere abspaltbare Schutzgruppe aufweisenden Aminosäure und/oder dem nachfolgend einzubauenden an seinem endständigen Stickstoffatom eine abspaltbare Schutzgruppe und, soweit erforderlich, an einem oder mehreren gegebenenfalls vorliegenden anderen Stickstoff- und/oder Sauerstoffatom(en) eine beziehungsweise mehrere Schutzgruppe(n) aufweisenden Peptidfragment beziehungsweise einem Esterderivat desselben vornimmt, wobei man so viele Kondensationen durchführt, wie es zum Einbau aller gewünschten Aminosäureeinheiten erforderlich ist, sowie danach in an sich bekannter Weise vom erhaltenen geschützten Peptidderivat die Schutzgruppe des endständigen Stickstoffatomes und die etwaige(n) Schutzgruppe(n) von anderen Stickstoffatomen beziehungsweise Sauerstoffatomen sowie gegebenenfalls die Aminogruppe der gegebenenfalls vorliegenden Aminooxygruppe selektiv schrittweise oder in einem Schritt entfernt, worauf man gegebenenfalls in an sich bekannter Weise das erhaltene Peptid der allgemeinen Formel I in ein Säureadditionssalz oder einen Komplex desselben überführt beziehungsweise gegebenenfalls das erhaltene Säureadditionssalz des Peptides der allgemeinen Formel I in ein anderes Säureadditionssalz oder in das Peptid der allgemeinen Formel I überführt.

2. Verfahren nach Anspruch 1, dadurch gekennzeichnet, dass man

a) zur Herstellung der Peptide der allgemeinen Formel I, bei welchen A für Wasserstoff steht, durch die Kondensation(en) als geschützte Peptide solche der allgemeinen Formel

B–X–Arg[C]–Val–Tyr[D]–Ile–His[E]–Pro–Y–O–F II,

worin

B für eine durch Acidolyse oder katalytische Hydrogenolyse entfernbare Schutzgruppe, insbesondere eine Benzyloxycarbonyl- oder tert.-Butyloxycarbonylgruppe, steht,

C eine Schutzgruppe zum zeitweiligen Schutz der Guanidinogruppe des Restes von Arginin, insbesondere eine Nitro- oder p-Toluolsulfonylgruppe, bedeutet,

D eine Schutzgruppe zum zeitweiligen Schutz der aromatischen Hydroxygruppe des Restes von Tyrosin, insbesondere eine, gegebenenfalls substituierte, Benzylgruppe, darstellt,

E für eine Schutzgruppe zum zeitweiligen Schutz der Imidazolgruppe des Restes von Histidin, insbesondere eine Dinitrophenylgruppe, steht,

F eine gegen die Einwirkung von milden Säuren widerstandsfähige, aber durch katalytische Hydrogenolyse oder durch die Einwirkung von stärkeren Säuren entfernbare Schutzgruppe zum Schutz der Carboxylgruppe des Restes der C-endständigen α-Hydroxycarbonsäure bedeutet und

X, Arg, Val, Tyr, Ile, His, Pro, Y und A wie oben festgelegt sind, aufbaut oder

b) zur Herstellung der Peptide der allgemeinen Formel I, bei welchen A einen Alkylrest mit 1 bis 5 Kohlenstoffatomen bedeutet, durch die Kondensation(en) als geschützte Peptide solche der allgemeinen Formel

B–X–Arg[C]–Val–Tyr[D]–Ile–His[E]–Pro–Y–O–A III

worin

B, C, D und E wie vorstehend festgelegt sind und

X, Arg, Val, Tyr, Ile, His, Pro, Y und A wie oben festgelegt sind, aufbaut.

3. Verfahren nach Anspruch 1 oder 2 zur Herstellung von Peptiden der allgemeinen Formel I, bei welchen X für einen Acylrest einer in der α-Stellung eine Aminooxygruppe aufweisenden aliphatischen Carbonsäure steht, dadurch gekennzeichnet, dass man durch die Kondensation(en) die entsprechenden geschützten Peptide der allgemeinen Formeln II beziehungsweise III, bei welchen X ebenfalls für einen Acylrest einer in der α-Stellung eine Aminooxygruppe aufweisenden aliphatischen Carbonsäure steht, aufbaut und das Abspalten der Schutzgruppe, für welche B steht, sowie gegebenenfalls der anderen durch Acidolyse abspaltbaren Schutzgruppen von ihnen durch Acidolyse, insbesondere durch Behandeln mit Fluorwasserstoffsäure, durchführt.

4. Verfahren nach Anspruch 1 oder 2 zur Herstellung von Peptiden der allgemeinen Formel I, bei welchen X für einen Acylrest einer in der α-Stellung eine Hydroxygruppe aufweisenden aliphatischen Carbonsäure steht, dadurch gekennzeichnet, dass man durch die Kondensation(en) die entsprechenden Peptide der allgemeinen Formeln II beziehungsweise III, bei welchen jedoch X für einen Acylrest einer in der α-Stellung eine Aminooxygruppe aufweisenden aliphatischen Carbonsäure steht, aufbaut und das Abspalten der Schutzgruppe, für welche B steht, sowie gegebenenfalls der anderen durch katalytische Hydrogenolyse abspaltbaren Schutzgruppen von ihnen durch katalytische Hydrierung unter gleichzeitiger Abspaltung der Aminogruppe der genannten α-Aminooxygruppe durchführt.

5. Verfahren nach Anspruch 1 bis 4, dadurch gekennzeichnet, dass man Peptide, bei welchen der Acylrest einer N-Methylaminosäure, für den X stehen kann, der Sarkosylrest ist, herstellt.

6. Verfahren nach Anspruch 1 bis 5, dadurch gekennzeichnet, dass man Peptide, bei welchen der Rest einer aliphatischen α-Hydroxycarbonsäure, für den Y steht, ein solcher mit einem asymmetrischen Kohlenstoffatom in der L-Konfiguration, insbesondere von L-Milchsäure oder L-2-Hydroxy-3-methylvaleriansäure, ist, herstellt.

7. Verfahren nach Anspruch 1 bis 6, dadurch gekennzeichnet, dass man Peptide, bei welchen der Alkylrest, für den A stehen kann, ein solcher mit 1 oder 2, ganz besonders 1, Kohlenstoffatom(en) ist, herstellt.

8. Verfahren nach Anspruch 1 bis 7, dadurch gekennzeichnet, dass man L-Sarkosyl-L-arginyl-L-valyl-L -tyrosyl-L -isoleucyl-L -histidyl-L-prolyl-L-2-hydroxy-3-methylvaleriansäure und ihre Alkylester mit 1 bis 5 Kohlenstoffatomen im Alkylteil herstellt.

9. Verfahren nach Anspruch 1 bis 7, dadurch gekennzeichnet, dass man L-Sarkosyl-L-arginyl-L-valyl- L-tyrosyl- L-isoleucyl- L-histidyl-L-prolyl-L-milchsäure und ihre Alkylester mit 1 bis 5 Kohlenstoffatomen im Alkylteil herstellt.

**Revendications pour les Etats contractants: BE, CH, LI, DE, FR, GB, IT, NL, SE**

1. Peptide de formule générale I ci-après:

X–Arg–Val–Tyr–Ile–His–Pro–Y–O–A. I,

dans laquelle:

X est un reste acyle d'un N-méthylamino-acide ou d'un acide carboxylique aliphatique comportant en position α- un groupe amino-oxy ou un groupe hydroxy-, chaque fois de configuration L- dans le cas d'un atome de carbone asymétrique,

Arg est un reste de L-arginine,

Val est un reste de L-valine,

Tyr est un reste de L-tyrosine,

Ile est un reste de L-isoleucine,

His est un reste de L-histidine,

Pro est un reste de L-proline,

Y est un reste d'un acide α-hydroxycarboxylique aliphatique dans lequel il manque dans son groupe hydroxy- un atome d'hydrogène par rapport à cet acide, et dans lequel le groupe carboxyle ne comporte par de groupe hydroxy-,

A représente un atome d'hydrogène où un reste alkyle comportant de 1 à 5 atomes de carbone,

ainsi que leurs sels d'addition d'acides et leurs complexes utilisables pharmaceutiquement.

2. Peptide selon la revendication 1, caractérisé en ce que le reste acyle d'un N-méthylamino-acide représenté par X, est un reste sarcosyle.

3. Peptide selon la revendication 1 ou 2, caractérisé en ce que le reste d'un acide α-hydroxycarboxylique aliphatique, représenté par Y, comporte un atome de carbone asymétrique en configuration L, en particulier le reste de l'acide L-lactique ou de l'acide L-2-hydroxy-3-méthylvalérianique.

4. Peptide selon l'une quelconque des revendications 1 à 3, caractérisé en ce que le reste alkyle, représenté par A, comporte 1 ou 2 et plus particulièrement 1, atome de carbone.

5. Acide L-sarcosyl- L-arginyl- L-valyl- L-tyrosyl- L-isoleucyl- L-histidyl- L-prolyl- L-2-hydroxy-3-méthylvalérianique et ses alkylesters comportant de 1 à 5 atomes de carbone dans la partie alkyle.

6. Acide- L-sarcosyl- L-arginyl- L-valyl- L-tyrosyl- L-isoleucyl- L-histidyl- L-prolyl- L-lactique et ses alkylesters comportant de 1 à 5 atomes de carbone dans la partie alkyle.

7. Procédé de préparation de composés selon l'une quelconque des revendications 1 à 6, caractérisé en ce que l'on condense d'une manière connue dans la chimie des Peptides, dans un acide α-hydroxycarboxylique aliphatique approprié ou dans un alkylester approprié de cet acide, comportant de 1 à 5 atomes de carbone dans la partie alkyle, ou dans un autre ester de cet acide, un amino-acide destiné à être incorporé ultérieurement dans ledit acide α-hydroxycarboxylique et comportant un groupe de protection séparable sur son atome d'azote terminal, lequel groupe de protection n'a qu'une fonction de protection du groupe ester, ledit amino-acide comportant, pour autant que cela est nécessaire, sur un autre atome d'azote ou sur un atome d'oxygène éventuellement existant, un autre groupe de protection séparable et/ou un fragment de peptide ou un dérivé ester dudit fragment de peptide, lequel fragment ou dérivé est destiné à être incorporé séquentiellement et comporte sur son atome d'azote terminal un groupe de protection séparable et, dans la mesure où cela est nécessaire, un ou plusieurs groupes de protection sur un ou plusieurs autres atomes d'azote et/ou d'oxygène, en ce que l'on réalise le cas échéant une nouvelle ou des nouvelles condensation(s) entre le produit intermédiaire peptidique obtenu, protégé sur son atome d'azote terminal et le ou les autre(s) produit(s) intermédiaire(s) peptidique(s) protégé(s) sur son ou leur atome d'azote terminal, obtenu(s) par d'éventuelles autres condensations du même type, après séparation du groupe de protection de l'atome d'azote terminal, d'une manière connue, ladite condensation étant effectuée avec, ou chaque fois avec, un amino-acide devant être incorporé séquentiellement et comportant un groupe de protection séparable sur son atome d'azote terminal et, dans la mesure où cela est nécessaire, comportant un autre groupe de protection séparable sur un autre atome d'azote ou sur un atome d'oxygène éventuellement existant, et/ou avec un fragment de peptide ou un dérivé ester de ce fragment, destiné à être incorporé séquentiellement et comportant un groupe de protection séparable sur son atome d'azote terminal et, dans la mesure où cela est nécessaire, comportant un ou plusieurs groupes de protection sur un ou plusieurs autres atomes d'azote ou d'oxygène éventuellement existants, et en ce que l'on réalise autant de condensations que cela est nécessaire pour incorporer toutes les unités d'amino-acides souhaités, et en ce que l'on élimine ensuite d'une manière connue, sélectivement progressivement ou en une seule étape, du dérivé peptidique protégé obtenu, le groupe de protection de l'atome d'azote terminal et le ou les groupe(s) de protection éventuel(s) des autres atomes d'azote ou des atomes d'oxygène, ainsi qu'éventuellement le groupe amino du groupe amino-oxy éventuellement existant, après quoi l'on transforme le cas échéant d'une manière connue, le peptide de formule générale I obtenu en un sel d'additions d'acide ou en un complexe de celui-ci où l'on transforme le cas échéant, le sel d'addition d'acide du peptide de formule générale I, obtenue en un autre sel d'additions d'acide ou en le peptide de formule générale I.

8. Procédé selon la revendication 7, caractérisé en ce que:

a) pour la préparation des peptides de formule générale I, dans lesquels A est l'hydrogène on synthétise par la ou les condensation(s) sous forme de peptides protégés, des peptides répondant à la formule générale II ci-après:

B–X–Arg(C)–Val–Tyr(D)–Ile–His(E)–Pro–Y–O–F II

dans laquelle:

B est un groupe de protection, en particulier un groupe benzyloxycarbonyle ou tert.-butyloxycarbonyle, séparable par acidolyse ou par hydrogenolyse catalytique,

C représente un groupe de protection pour la protection temporaire du groupe guanidine du reste arginine, en particulier un groupe nitro- ou p-toluènesulfonyle,

D représente un groupe de protection pour la protection temporaire du groupe hydroxy aromatique du reste tyrosine, en particulier un groupe benzyle, éventuellement substitué,

E est un groupe de protection pour la protection temporaire du groupe imidazole du reste histidine, en particulier un groupe dinitrophenyle,

F représente un groupe de protection résistant à l'action des acides doux, mais éliminable par hydrogénolyse catalytique ou par action d'acides forts, lequel groupe de protection sert à la protection du groupe carboxyle du reste de l'acide α-hydroxycarboxylique qui se trouve sur le carbone terminal, et

X, Arg, Val, Tyr, Ile, His, Pro, Y et A sont tels que définis dans les revendications 1 à 4, ou

b) pour la préparation des peptides de formule générale I, dans lesquels A représente un reste alkyle comportant de 1 à 5 atomes de carbone on synthétise, par la ou les condensation(s) sous forme de peptides protégés, des peptides répondant à la formule générale III ci-après:

B–X–Arg(C)–Val–Tyr(D)–Ile–His(E)–Pro–Y–O–A III

dans laquelle:

B, C, D et E sont tels que définis ci-dessus et

X, Arg, Val, Tyr, Ile, His, Pro, Y et A sont tels que définis dans les revendications 1 à 4.

9. Procédé selon la revendication 7 ou 8 pour la préparation de peptides de la formule générale I, dans lesquels X est un reste acyle d'un acide carboxylique aliphatique comportant un groupe amino-oxy- en position α-, caractérisé en ce que l'on synthétise par condensation les peptides protégés correspondants des formules générales II et III, dans lesquels X est également un reste acyle d'un acide carboxylique aliphatique comportant un groupe amino-oxy- en position α- et l'on effectue la séparation du groupe de protection, représenté par B, ainsi qu'éventuellement des autres groupes de protection séparables, par acidolyse, en particulier par traitement par l'acide fluorhydrique.

10. Procédé selon la revendication 7 ou 8 pour la préparation de peptides de formule générale I, dans lesquels X représente un reste acyle d'un acide carboxylique aliphatique comportant un groupe hydroxy- en position α, caractérisé en ce que l'on synthétise par condensation(s), les peptides correspondants des formules générales II ou III, dans lesquels cependant X est un reste acyle d'un acide carboxylique aliphatique comportant un groupe amino-oxy- en position α- et l'on effectue la séparation du groupe de protection, représenté par B, ainsi qu'éventuellement des autres groupes de protection séparables de ces peptides par hydrogenolyse catalytique au moyen d'une

hydrogénation catalytique réalisée simultanément avec la séparation du groupe amino du groupe α-amino-oxy précité.

11. Médicaments ou agents de diagnostic caractérisés en ce qu'ils contiennent un ou plusieurs composés selon l'une quelconque des revendications 1 à 6, en tant que constituant(s) actif(s), avantageusement conjointement avec les agents de formulation pharmaceutiques usuels.

**Revendications pour l'Etat contractant AT**

1. Procédé de préparation de peptides de formule générale I ci-après:

X–Arg–Val–Tyr–Ile–His–Pro–Y–O–A. I,

da laquelle:

X est un reste acyle d'un N-méthylamino-acide ou d'un acide carboxylique aliphatique comportant en position α- un groupe amino-oxy ou un groupe hydroxy-, chaque fois de configuration L- dans le cas d'un atome de carbone asymétrique,

Arg est un reste de L-arginine,

Val est un reste de L-valine,

Tyr est un reste de L-tyrosine,

Ile est un reste de L-isoleucine,

His est un reste de L-histidine,

Pro est un reste de L-proline,

Y est un reste d'un acide α-hydroxycarboxylique aliphatique dans lequel il manque dans son groupe hydroxy- un atome d'hydrogène par rapport à cet acide, et dans lequel le groupe carboxyle ne comporte pas de groupe hydroxy-,

A représente un atome d'hydrogène où un reste alkyle comportant de 1 à 5 atomes de carbone,

ainsi que de leurs sels d'addition d'acides et leurs complexes utilisables pharmaceutiquement, caractérisé en ce que l'on condense d'une manière connue dans la chimie des Peptides, dans un acide α-hydroxycarboxylique aliphatique approprié ou dans un alkylester approprié de cet acide, comportant de 1 à 5 atomes de carbone dans la partie alkyle, ou dans un autre ester de cet acide, un amino-acide destiné à être incorporé ultérieurement dans ledit acide α-hydroxycarboxylique et comportant un groupe de protection séparable sur son atome d'azote terminal, lequel groupe de protection n'a qu'une fonction de protection du groupe ester, ledit amino-acide comportant, pour autant que cela est nécessaire, sur un autre atome d'azote ou sur un atome d'oxygène éventuellement existant, un autre groupe de protection séparable et/ou un fragment de peptide ou un dérivé ester dudit fragment de peptide, lequel fragment ou dérivé est destiné à être incorporé séquentiellement et comporte sur son atome d'azote terminal un groupe de protection séparable et, dans la mesure où cela est nécessaire, un ou plusieurs groupes de protection sur un ou plusieurs autres atomes d'azote et/ou d'oxygène, en ce que l'on réalise le cas échéant une nouvelle ou des nouvelles condensation(s) entre le produit intermédiaire peptidique obtenu, protégé sur son atome d'azote terminal et le ou les autre(s) produit(s) intermédiaire(s) peptideque(s) protégé(s) sur son ou leur atome d'azote terminal, obtenu(s) par d'éventuelles autres condensations du même type, après séparation du groupe de protection de l'atome d'azote terminal, d'une manière connue, ladite condensation étant effectuée avec, ou chaque fois avec, un amino-acide devant être incorporé séquentiellement et comportant un groupe de protection séparable sur son atome d'azote terminal et, dans la mesure où cela est nécessaire, comportant un autre gorupe de protection séparable sur un autre atome d'azote ou sur un atome d'oxygène éventuellement existant, et/ou avec un fragment de peptide ou un dérivé ester de ce fragment, destiné à être incorporé séquentiellement et comportant un groupe de protection séparable sur son atome d'azote terminal et, dans la mesure où cela est nécessaire, comportant un ou plusieurs groupes de protection sur un ou plusieurs autres atomes d'azote ou d'oxygène éventuellement existants, et en ce que l'on réalise autant de condensations que cela est nécessaire pour incorporer toutes les unités d'amino-acides souhaités, et en ce que l'on élimine ensuite d'une manière connue, sélectivement progressivement ou en une seule étape, du dérivé peptidique protégé obtenu, le groupe de protection de l'atome d'azote terminal et le ou les groupe(s) de protection éventuel(s) des autres atomes d'azote ou des atomes d'oxygène, ainsi qu'éventuellement le groupe amino du groupe amino-oxy éventuellement existant, après quoi l'on transforme le cas échéant d'une manière connue, le peptide de formule générale I obtenu en un sel d'additions d'acide ou en un complexe de celui-ci où l'on transforme le cas échéant, le sel d'addition d'acide du peptide de formule générale I, obtenu en un autre sel d'additions d'acide ou en le peptide de formule générale I.

2. Procédé selon la revendication 1, caractérisé en ce que:

a) pour la préparation des peptides de formule générale I, dans lesquels A est l'hydrogène on synthétise par la ou les condensation(s) sous forme de peptides protégés, des peptides répondant à la formule générale II ci-après:

B–X–Arg(C)–Val–Tyr(D)–Ile–His(E)–Pro–Y–O–F II

dans laquelle:

B est un groupe de protection, en particulier un groupe benzyloxycarbonyle ou tert.-butyloxycarbonyle, séparable par acidolyse ou par hydrogenolyse catalytique,

C représente un groupe de protection pour la protection temporaire du groupe guanidine du reste arginine, en particulier un groupe nitro- ou p-toluènesulfonyle,

D représente un groupe de protection pour la protection temporaire du groupe hydroxy aromatique du reste tyrosine, en particulier un groupe benzyle, éventuellement substitué,

E est un groupe de protection pour la protection temporaire du groupe imidazole du reste histidine, en particulier un groupe dinitrophenyle,

F représente un groupe de protection résistant à l'action des acides doux, mais éliminable par hydrogénolyse catalytique ou par action d'acides forts, lequel groupe de protection sert à la protection du groupe carboxyle du reste de l'acide α-hydroxycarboxylique qui se trouve sur le carbone terminal, et

X, Arg, Val, Tyr, Ile, His, Pro, Y et A sont tels que définis ci-dessus ou

b) pour la préparation des peptides de formule générale I, dans lesquels A représente un reste alkyle comportant de 1 à 5 atomes de carbone on synthétise, par la ou les condensation(s) sous forme de peptides protégés, des peptides répondant à la formule générale III ci-après:

B–X–Arg(C)–Val–Tyr(D)–Ile–His(E)–Pro–Y–O–A III

dans laquelle:

B, C, D et E sont tels que définis dessus et

X, Arg, Val, Tyr, Ile, His, Pro, Y et A sont tels que définis ci-dessus.

3. Procédé selon la revendication 1 ou 2 pour la préparation de peptides de la formule générale I, dans lesquels X est un reste acyle d'un acide carboxylique aliphatique comportant un groupe amino-oxy- en position α-, caractérisé en ce que l'on synthétise par condensation les peptides protégés correspondants des formules générales II et III, dans lesquels X est également un reste acyle d'un acide carboxylique aliphatique comportant un groupe amino-oxy- en position α- et l'on effectue la séparation du groupe de protection, représenté par B, ainsi qu'éventuellement des autres groupes de protection séparables, par acidolyse, en particulier par traitement par l'acide fluorhydrique.

4. Procédé selon la revendication 1 ou 2 pour la préparation de peptides de formule générale I, dans lesquels X représente un reste acyle d'un acide carboxylique aliphatique comportant un groupe hydroxy- en position α, caractérisé en ce que l'on synthétise par condensation(s), les peptides correspondants des formules générales II ou III, dans lesquels cependant X est un reste acyle d'un acide carboxylique aliphatique comportant un groupe amino-oxy- en position α- et l'on effectue la séparation du groupe de protection, représenté par B, ainsi qu'éventuellement des autres groupes de protection séparables de ces peptides par hydrogenolyse catalytique au moyen d'une hydrogénation catalytique réalisée simultanément avec la séparation du groupe amino du groupe α-amino-oxy précité.

5. Procédé selon les revendications 1 à 4, caractérisé en ce que l'on prépare peptides dans lesquelles le reste acyle d'un N-méthylamino-acide représenté par X, est un reste sarcosyle.

6. Procédé selon les revendications 1 à 5, caractérisé en ce que l'on prépare peptides dans lesquelles le reste d'un acide α-hydroxycarboxylique aliphatique, représenté par Y, comporte un atome de carbone asymétrique en configuration L, en particulier le reste de l'acide L-lactique ou de l'acide L-2-hydroxy-3-méthylvalérianique.

7. Procédé selon les revendications 1 à 6, caractérisé en ce que l'on prépare peptides dans lesquelles le reste alkyle, représenté par A, comporte 1 ou 2 et plus particulièrement 1, atome de carbone.

8. Procédé selon les revendications 1 à 7, caractérisé en ce que l'on prépare l'cide L-sarcosyl- L-arginyl- L-valyl- L-tyrosyl- L-isoleucyl- L-histidyl- L-prolyl- L-2-hydroxy-3-méthylvalérianique et ses alkylesters comportant de 1 à 5 atomes de carbone dans la partie alkyle.

9. Procédé selon les revendications 1 à 7, caractérisé en ce que l'on prépare l'acide- L-sarcosyl- L-arginyl- L-valyl- L-tyrosyl- L-isoleucyl- L-histidyl- L-prolyl- L-lactique et ses alkylesters comportant de 1 à 5 atomes de carbone dans la partie alkyle.

**Claims for the contracting staates: BE, CH, LI, DE, FR, GB, IT, NL, SE,**

1. Peptides having the general formula

X–Arg–Val–Tyr–Ile–His–Pro–Y–O–A I,

wherein

X represents an acyl radical of a N-methylaminoacid or of an aliphatic carboxylic acid having in the α-position an aminooxy group or a hydroxyl group, each in the L-configuration in case of an asymmetrical carbon atom,

Arg represents a radical of L-arginine,

Val represents a radical of L-valine,

Tyr represents a radical of L-tyrosine,

Ile represents a radical of L-isoleucine,

His represents a radical of L-histidine,

Pro represents a radical of L-proline,

Y represents a radical of an aliphatic α-hydroxycarboxylic acid having 1 lacking hydrogen atom of its hydroxyl group with respect of this acid beyond the absence of the hydroxyl group of the carboxylic group and

A represents hydrogen or an alkyl radical having from 1 to 5 carbon atom(s),

as well as their acid addition salts and pharmaceutically useful complexes.

2. Peptides according to claim 1, characterized in that the acyl radical of a N-methylaminoacid which can be represented by X is the sarcosyl radical.

3. Peptides according to claim 1 or 2, characterized in that the radical of an aliphatic α-hydroxycarboxylic acid which can be represented by Y is such having an asymmetrical carbon atom in the L-configuration, particularly of L-lactic acid or L-2-hydroxy-3-methylvaleric acid.

4. Peptides according to claims 1 to 3, characterized in that the alkyl radical which can be represented by A is such having 1 or 2, more particularly 1, carbon atom(s).

5. L-sarcosyl- L-arginyl- L-valyl- L-tyrosyl- L-isoleucyl- L-histidyl- L-prolyl- L-2-hydroxy-3-methylvaleric acid and its alkyl esters having from 1 to 5 carbon atoms in the alkyl part.

6. L-sarcosyl- L-arginyl- L-valyl- L-tyrosyl-

L-isoleucyl- L-histidyl- L-prolyl- L-lactic acid and its alkyl esters having from 1 to 5 carbon atoms in the alkyl part.

7. A process for the preparation of the compounds according to claims 1 to 6, characterized in that one condenses in a manner known per se in the peptide chemistry a propper aliphatic α-hydroxyl carboxylic acid or a propper alkyl ester of it having from 1 to 5 carbon atoms in the alkyl part or another ester of it only with a protective function of the ester group with the amino acid to be incorporated successively and having on its terminal nitrogen atom a splittable-off protective group and, as far as necessary, on another nitrogen atom being optionally present another splittable-off protective group and/or with the peptide fragment or an ester derivative of it, respectively, successively to be incorporated and having on its terminal nitrogen atom a splittable-off protective group and, as far as necessary, on one or several other nitrogen and/or oxygen atom(s) one or several protective group(s) and, if desired, one carries out with the obtained peptide intermediate protected on the terminal nitrogen atom and with the further peptide intermediate(s) obtained by possible further condensations of this type and protected on the terminal nitrogen atom after having removed the protective group of the terminal nitrogen atom one further condensation or further condensations, respectively, with the amino acid or with the respective amino acid, respectively, successively to be incorporated and having on its terminal nitrogen atom a splittable-off protective group and, as far as necessary, on another nitrogen atom or oxygen atom optionally present another splittable-off protective group and/or with the peptide fragment or an ester derivative of it, respectively, successively to be incorporated and having on its terminal nitrogen atom a splittable-off protective group and, as far as necessary, on one or several other nitrogen and/or oxygen atom(s) optionally present one or several protective group(s) accomplishing as much condensations as are necessary for incorporating all desired amino acid units as well as thereafter in a manner known per se selectively step by step or in one step one removes from the obtained protected peptide derivative the protective group of the terminal nitrogen atom and the possible protective group(s) of other nitrogen atoms or oxygen atoms, respectively, as well as, if desired, the amino group of the aminooxy group optionally present, whereafter, if desired, in a manner known per se one converts the obtained peptide of the general formula I into an acid addition salt or into a complex of it or, if desired, the obtained acid addition salt of the peptide of the general formula I into another acid addition salt or in the peptide of the general formula I, respectively.

8. A process according to claim 7, characterized in that

a) for the preparation of the peptides of the general formula I in which A represents hydrogen one synthetizes by the condensation(s) as protected peptides such having the general formula

B–X–Arg[C]–Val–Tyr[D]–Ile–His[E]–Pro–Y–O–F II,

wherein

B represents a protective group removable by acidolysis or catalytic hydrogenolysis, particularly a benzyloxycarbonyl- or tert.-butyloxycarbonyl group,

C means a protective group for the temporary protection of the guanidino group of the radical of arginine, particularly a nitro or p-toluenesulfonyl group,

D represents a protective group for the temporary protection of the aromatic hydroxyl group of the radical of tyrosine, particularly a benzyl group, optionally substituted,

E is a protective group for the temporary protection of the imidazole group of the radical of histidine, particularly a dinitrophenyl group,

F means a protective group resistant to the action of mild acids but removable by catalytic hydrogenolysis or by the action of stronger acids for the protection of the carboxyl group of the radical of the C-terminal α-hydroxylcarboxylic acid and

X, Arg, Val, Tyr, Ile, His, Pro, Y and A are as defined in claims 1 to 4 or

b) for the preparation of the peptides of the general formula I in which A means an alkyl radical having from 1 to 5 carbon atoms one synthetizes by the condensation(s) as protected peptides such having the general formula

B–X–Arg[C]–Val–Tyr[D]–Ile–His[E]–Pro–Y–O–A III,

wherein

B, C, D and E are as defined in the foregoing and

X, Arg, Val, Tyr, Ile, His, Pro, Y and A are as defined in claims 1 to 4.

9. A process according to claim 7 or 8 for the preparation of peptides of the general formula I in which X represents an acyl radical of an aliphatic carboxylic acid having in the α-position an aminooxy group, characterized in that one synthetizes by the condensation(s) the corresponding protective peptides of the general formulae II or III, respectively, in which X likewise represents an acyl radical of an aliphatic carboxylic acid having an aminooxy group in the α-position and one carries out the splitting-off of the protective group represented by B as well as, if desired, of the other protective groups splittable-off by acidolysis from them by acidolysis, particularly by treatment with hydrofluoric acid.

10. A process according to claim 7 or 8 for the preparation of peptides of the general formula I in which X represents an acyl radical of an aliphatic carboxylic acid having a hydroxyl group in the α-position, characterized in that one synthetizes by the condensation(s) the corresponding peptides of the general formulae II or III, respectively, in which, however, X represents an acyl radical of an aliphatic carboxylic acid having an aminooxy group in the α-position and one carries out the

splitting-off of the protective group represented by B as well, if desired, of the other protective groups splittable-off by catalytic hydrogenolysis from them by catalytic hydrogenation with simultaneous splitting-off of the amino group of the said α-aminooxy group.

11. Medicaments or diagnostic agents, characterized by a content of 1 or more compounds according to claims 1 to 6 as an active principle, or active principles, respectively, usefully together with usual pharmaceutical processing agents.

**Claims for the contracting staate AT**

1. A process for the preparation of peptides having the general formula

X–Arg–Val–Tyr–Ile–His–Pro–Y–O–A I,

wherein

X represents an acyl radical of a N-methylaminoacid or of an aliphatic carboxylic acid having in the α-position an aminooxy group or a hydroxyl group, each in the L-configuration in case of an asymmetrical carbon atom,

Arg represents a radical of L-arginine,

Val represents a radical of L-valine,

Tyr represents a radical of L-tyrosine,

Ile represents a radical of L-isoleucine,

His represents a radical of L-histidine,

Pro represents a radical of L-proline,

Y represents a radical of an aliphatic α-hydroxycarboxylic acid having 1 lacking hydrogen atom of its hydroxyl group with respect of this acid beyond the absence of the hydroxyl group of the carboxylic group and

A represents hydrogen or an alkyl radical having from 1 to 5 carbon atom(s),

as well as of their acid addition salts and pharmaceutically useful complexes, characterized in that one condenses in a manner known per se in the peptide chemistry a propper aliphatic α-hydroxyl carboxylic acid or a propper alkyl ester of it having from 1 to 5 carbon atoms in the alkyl part or another ester of it only with a protective function of the ester group with the amino acid to be incorporated successively and having on its terminal nitrogen atom a splittable-off protective group and, as far as necessary, on another nitrogen atom being optionally present another splittable-off protective group and/or with the peptide fragment or an ester derivative of it, respectively, successively to be incorporated and having on its terminal nitrogen atom a splittable-off protective group and, as far as necessary, on one or several other nitrogen and/or oxygen atom(s) one or several protective group(s) and, if desired, one carries out with the obtained peptide intermediate protected on the terminal nitrogen atom and with the further peptide intermediate(s) obtained by possible further condensations of this type and protected on the terminal nitrogen atom after having removed the protective group of the terminal nitrogen atom one further condensation or further condensations, respectively, with the amino acid or with the respective amino acid, respectively, successively to be incorporated and having on its terminal nitrogen atom a splittable-off protective group and, as far as necessary, on another nitrogen atom or oxygen atom optionally present another splittable-off protective group and/or with the peptide fragment or an ester derivative of it, respectively, successively to be incorporated and having on its terminal nitrogen atom a splittable-off protective group and, as far as necessary, on one or several other nitrogen and/or oxygen atom(s) optionally present one or several protective group(s) accomplishing as much condensations as are necessary for incorporating all desired amino acid units as well as thereafter in a manner known per se selectively step by step or in one step one removes from the obtained protected peptide derivative the protective group of the terminal nitrogen atom and the possible protective group(s) of other nitrogen atoms or oxygen atoms, respectively, as well as, if desired, the amino group of the aminooxy group optionally present, whereafter, if desired, in a manner known per se one converts the obtained peptide of the general formula I into an acid addition salt or into a complex of it or, if desired, the obtained acid addition salt of the peptide of the general formula I into another acid addition salt or in the peptide of the general formula I, respectively.

2. A process according to claim 1, characterized in that

a) for the preparation of the peptides of the general formula I in which A represents hydrogen one synthetizes by the condensation(s) as protected peptides such having the general formula

B–X–Arg[C]–Val–Tyr[D]–Ile–His[E]–Pro–Y–O–F II,

wherein

B represents a protective group removable by acidolysis or catalytic hydrogenolysis, particularly a benzyloxycarbonyl- or tert.-butyloxycarbonyl group,

C means a protective group for the temporary protection of the guanidino group of the radical of arginine, particularly a nitro or p-toluenesulfonyl group,

D represents a protective group for the temporary protection of the aromatic hydroxyl group of the radical of tyrosine, particularly a benzyl group, optionally substituted,

E is a protective group for the temporary protection of the imidazole group of the radical of histidine, particularly a dinitrophenyl group,

F means a protective group resistant to the action of mild acids but removable by catalytic hydrogenolysis or by the action of stronger acids for the protection of the carboxyl group of the radical of the C-terminal α-hydroxylcarboxylic acid and

X, Arg, Val, Tyr, Ile, His, Pro, Y and A are as above defined or

b) for the preparation of the peptides of the general formula I in which A means an alkyl radical having from 1 to 5 carbon atoms one synthetizes by the condensation(s) as protected peptides such having the general formula

B–X–Arg[C]–Val–Tyr[D]–Ile–His[E]–Pro–Y–O–A III,

wherein

B, C, D and E are as defined in the foregoing and

X, Arg, Val, Tyr, Ile, His, Pro, Y and A are as above defined.

3. A process according to claim 1 or 2 for the preparation of peptides of the general formula I in which X represents an acyl radical of an aliphatic carboxylic acid having in the α-position an aminooxy group, characterized in that one synthetizes by the condensation(s) the corresponding protective peptides of the general formulae II or III, respectively, in which X likewise represents an acyl radical of an aliphatic carboxylic acid having an aminooxy group in the α-position and one carries out the splitting-off of the protective group represented by B as well as, if desired, of the other protective groups splittable-off by acidolysis from them by acidolysis, particularly by treatment with hydrofluoric acid.

4. A process according to claim 1 or 2 for the preparation of peptides of the general formula I in which X represents an acyl radical of an aliphatic carboxylic acid having a hydroxyl group in the α-position, characterized in that one synthetizes by the condensation(s) the corresponding peptides of the general formulae II or III, respectively, in which, however, X represents an acyl radical of an aliphatic carboxylic acid having an aminooxy group in the α-position and one carries out the splitting-off of the protective group represented by B as well, if desired, of the other protective groups splittable-off by catalytic hydrogenolysis from them by catalytic hydrogenation with simultaneous splitting-off of the amino group of the said α-aminooxy group.

5. A process according to claims 1 to 4, characterized in that peptides in which the acyl radical of a N-methylaminoacid which can be represented by X is the sarcosyl radical are prepared.

6. A process according to claims 1 to 5, characterized in that peptides in which the radical of an aliphatic α-hydroxycarboxylic acid which can be represented by Y is such having an asymmetrical carbon atom in the L-configuration, particularly of L-lactic acid or L-2-hydroxy-3-methylvaleric acid are prepared.

7. A process according to claims 1 to 6, characterized in that peptides in which the alkyl radical which can be represented by A is such having 1 or 2, more particularly 1, carbon atom(s) are prepared.

8. A process according to claims 1 to 7, characterized in that L-sarcosyl- L-arginyl- L-valyl- L-tyrosyl- L-isoleucyl- L-histidyl- L-propyl- L-2-hydroxy-3-methylvaleric acid and its alkyl esters having from 1 to 5 carbon atoms in the alkyl part are prepared.

9. A process according to claims 1 to 7, characterized in that L-sarcosyl- L-arginyl- L-valyl- L-tyrosyl- L-isoleucyl- L-histidyl- L-prolyl- L-lactic acid and its alkyl esters having from 1 to 5 carbon atoms in the alkyl part are prepared.